(19) Europäisches Patentamt
European Patent Office
Office européen des brevets

(11) **EP 3 156 059 B1**

(12) **FASCICULE DE BREVET EUROPEEN**

(45) Date de publication et mention de la délivrance du brevet:
**06.06.2018 Bulletin 2018/23**

(21) Numéro de dépôt: **16203835.0**

(22) Date de dépôt: **31.07.2015**

(51) Int Cl.:
*A61K 31/722* (2006.01)   *A61K 9/00* (2006.01)
*A61K 47/10* (2017.01)   *A61K 47/26* (2006.01)
*A61K 9/06* (2006.01)   *A61P 19/02* (2006.01)
*A61L 27/20* (2006.01)   *A61L 27/50* (2006.01)

(54) **COMPOSITION THERMOGÉLIFIABLE STÉRILISÉE**

STERILISIERTE THERMOGELIFIZIERBARE ZUSAMMENSETZUNG

STERILISED THERMOGELLING COMPOSITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.08.2014 FR 1457546**

(43) Date de publication de la demande:
**19.04.2017 Bulletin 2017/16**

(62) Numéro(s) de document de la (des) demande(s) initiale(s) en application de l'article 76 CBE:
**15742326.0 / 3 016 663**

(73) Titulaire: **Kiomed Pharma**
**4040 Herstal (BE)**

(72) Inventeurs:
• **CHAUSSON, Mickaël**
**4520 WANZE (BE)**
• **LECLER, Renaud**
**4102 OUGREE (BE)**

(74) Mandataire: **Lavoix**
**2, place d'Estienne d'Orves**
**75441 Paris Cedex 09 (FR)**

(56) Documents cités:
**US-A1- 2013 244 972**

• YUHUA CHANG ET AL: "Preparation and properties of a novel thermosensitive N-trimethyl chitosan hydrogel", POLYMER BULLETIN, SPRINGER, BERLIN, DE, vol. 63, no. 4, 13 mai 2009 (2009-05-13), pages 531-545, XP019758287, ISSN: 1436-2449, DOI: 10.1007/S00289-009-0103-6

• WU J ET AL: "A thermo- and pH-sensitive hydrogel composed of quaternized chitosan/glycerophosphate", INTERNATIONAL JOURNAL OF PHARMACEUTICS, ELSEVIER BV, NL, vol. 315, no. 1-2, 6 juin 2006 (2006-06-06), pages 1-11, XP027972330, ISSN: 0378-5173 [extrait le 2006-06-06]

• KHOR E ET AL: "Implantable applications of chitin and chitosan", BIOMATERIALS, ELSEVIER SCIENCE PUBLISHERS BV., BARKING, GB, vol. 24, no. 13, 1 juin 2003 (2003-06-01), pages 2339-2349, XP004420026, ISSN: 0142-9612, DOI: 10.1016/S0142-9612(03)00026-7

• ZANG SHENGQI ET AL: "A comparison of physicochemical properties of sterilized chitosan hydrogel and its applicability in a canine model of periodontal regeneration", CARBOHYDRATE POLYMERS, vol. 113, 16 juillet 2014 (2014-07-16), pages 240-248, XP029072731, ISSN: 0144-8617, DOI: 10.1016/J.CARBPOL.2014.07.018

• JARRY CLAIRE ET AL: "Irradiating or autoclaving chitosan/polyol solutions: effect on thermogelling chitosan-beta-glycerophosphate systems.", CHEMICAL & PHARMACEUTICAL BULLETIN OCT 2002, vol. 50, no. 10, octobre 2002 (2002-10), pages 1335-1340, XP002737864, ISSN: 0009-2363

• CHENGDONG JI ET AL: "Sterilization-free chitosan hydrogels for controlled drug release", MATERIALS LETTERS, NORTH HOLLAND PUBLISHING COMPANY. AMSTERDAM, NL, vol. 72, 28 décembre 2011 (2011-12-28), pages 110-112, XP028398000, ISSN: 0167-577X, DOI: 10.1016/J.MATLET.2011.12.116 [extrait le 2012-01-03]

**Description**

La présente invention concerne une composition thermogélifiable stérilisée. Plus particulièrement, la présente invention concerne une telle composition stérilisée à la vapeur, et un procédé de stérilisation pour préparer une telle composition, et leurs utilisations, notamment dans le domaine médical ou pour une injection dans le corps humain ou animal.

Il est préconisé de stériliser les appareillages, échantillons, et autres dispositifs qui doivent être en contact avec le corps humain ou animal. Pour réaliser de manière industrielle une telle stérilisation, il est avantageux de stériliser à la vapeur.

Cependant, dans un certain nombre de cas spécifiques, la stérilisation à la vapeur ne convient pas. Il s'agit notamment des cas dans lesquels les éléments à stériliser ne supportent pas une élévation en température, la présence de vapeur d'eau, ou encore si l'élément stérilisé est modifié par les conditions de stérilisation de sorte qu'il ne soit plus convenable pour son utilisation dans l'application visée.

Il est connu du brevet EP 2 361 640 que les compositions thermogélifiable à base de chitosane ne sont pas stérilisables à la vapeur, en particulier car une telle stérilisation influence la structure et les propriétés fonctionnelles de l'hydrogel, et ainsi la capacité de la composition à gélifier ou à revenir sous sa forme initiale, fluide. Il est donc déconseillé de réaliser une telle stérilisation à la vapeur pour stériliser un hydrogel de chitosane et ses dérivés. Jarry et al. (Effects of steam sterilization on thermogelling chitosan-based gels. J Biomed Mater Res (Appl Biomater), 2011, 58, 127-135) enseignent de stériliser au préalable une solution de chitosane puis d'ajouter les autres composés de l'hydrogel, comme le beta glycérol phosphate de sodium, eux aussi stérilisés séparément. Cette procédure de stérilisation n'intervient pas sur le produit final, et impose de réaliser le mélange des solutions de manière aseptique, ce qui n'est pas avantageux d'un point de vue industriel. Il est encore déconseillé dans le brevet US 6 344 488 de stériliser à la vapeur une composition thermogélifiable de chitosane puisque le gel est thermosensible. La solution de chitosane est ainsi stérilisée à la vapeur avant ajout de l'agent gélifiant. Chengdong Ji et al. ont décrit la stérilisation de chitosane en présence de glycérol phosphate (Sterilization-free chitosan hydrogels for controlled drug release, Materials Letters, 2012, 72,110-112), mais aucune propriété mécanique n'a été étudiée. Il n'est pas possible de connaître si des gels de chitosane ainsi stérilisés sont utilisables autrement que comme vecteur de médicaments, notamment lorsque des propriétés rhéologiques et/ou mécaniques particulières sont recherchées. Jarry et al. (Irradiating or autoclaving chitosan/polyol solutions: effect on thermogelling chitosan-$\beta$-glycerophosphate systems, Chem Pharm Bull, 2002, 50(10), 1335 -1340) ont aussi étudié l'effet de la stérilisation à la vapeur de gels de chitosane. Ils suggèrent un pouvoir bénéfique de certains polyols. Les pH de telles compositions thermogélifiables sont inférieurs à 7,2. Enfin, le brevet US 6 344 488 supporte que la transition sol-gel de telles compositions thermogélifiantes (« thermogels » de chitosane est irréversible à pH supérieur à 6,9. Ces thermogels dont la transition sol-gel est irréversible présentent l'inconvénient en pratique de ne pas pouvoir être stérilisés par augmentation de la température ou à la vapeur afin que les compositions ne gélifient pas avant leur utilisation. Il s'agit donc d'un inconvénient majeur.

L'invention a pour but de résoudre ces problèmes techniques.

Il existe en particulier le besoin de fournir une composition stérile thermogélifiable, gélifiée à température physiologique d'un être humain ou animal, présentant de bonnes propriétés physico-chimiques pour des applications thérapeutiques ou esthétiques.

L'invention a notamment pour but de fournir une composition thermogélifiable stérile qui puisse être facilement injectée tout en étant gélifiée à température physiologique d'un être humain. Une telle composition stérile doit présenter de bonnes propriétés physico-chimiques pour des applications thérapeutiques ou esthétiques, en particulier à la température physiologique de 37°C. Plus particulièrement, une telle composition stérile devrait pouvoir présenter des propriétés viscoélastiques modulables selon l'application visée pour se rapprocher le plus précisément possible des propriétés nécessaires pour le but thérapeutique ou esthétique visé.

L'invention a pour but également de fournir une composition stérile thermogélifiable dont le pH soit acceptable pour les applications thérapeutiques ou esthétiques visées. Plus particulièrement, de telles compositions stériles devraient pouvoir présenter un pH modulable selon l'application visée, notamment pour être le plus proche possible du pH physiologique.

La présente invention a également pour but de fournir une composition stérile thermogélifiable dont l'osmolarité est proche de celle physiologique pour l'application thérapeutique ou esthétique visée, en particulier une osmolalité de 100 à 700 mosm/kg, et en particulier de 200 à 500mosm/kg (écrit aussi « mosmol/kg »).

La présente invention a pour but également selon une variante de fournir une composition stérile dont la transition sol-gel soit réversible, notamment afin que les conditions de stockage et/ou de transport ne nécessitent pas un contrôle drastique de la température, et/ou que la composition puisse subir une stérilisation à la vapeur, voire plusieurs cycles de stérilisation à la vapeur.

L'invention a encore pour but de fournir une composition utilisable à titre de viscosupplément, et en particulier de pouvoir être injectée dans ou en mélange avec un fluide synovial. L'invention a notamment pour but de fournir un fluide synovial reconstruit, c'est-à-dire une composition restaurant les propriétés d'une articulation, en particulier en lui apportant une capacité à absorber les chocs lors d'un mouvement et à être lubrifiée au moins au repos.

L'invention a encore pour but de fournir une composition présentant de bonnes propriétés en mélange avec un fluide synovial, et en particulier un fluide synovial d'un être humain. Avantageusement, l'invention a pour but de fournir une composition présentant une bonne résistance à la dégradation dans un environnement inflammatoire, en particulier en termes de conservation du module d'élasticité G' et de la viscosité au repos.

La présente invention permet de manière surprenante de résoudre un ou plusieurs problèmes énoncés ci-dessus.

Il a été découvert de manière surprenante qu'une telle composition pouvait être préparée lorsque cette composition comprend un chitosane présentant des unités N-acétyl-glucosamine, des unités glucosamine, et des unités glucosamine substituées autres que les unités N-acétyl-glucosamine, ledit chitosane substitué présentant de préférence un degré de substitution des unités glucosamine allant de 10 à 50%, exprimé en nombre de mole du substituant par rapport au nombre de mole d'unités totales.

Selon un mode de réalisation particulier, les unités glucosamine sont des unités D-glucosamine (unités D-glucosamine, unités D-glucosamine substituées, unités N-acétyl-D-glucosamine et éventuellement des unités N-acétyl-D-glucosamine substituées).

Selon une variante, le chitosane présente des unités N-acétyl-D-glucosamine, des unités D-glucosamine, et des unités D-glucosamine substituées autres que les unités N-acétyl-D-glucosamine, et éventuellement des unités N-acétyl-D-glucosamine substituées.

Par « D-glucosamine substituées autres que les unités N-acétyl-D-glucosamine », on entend que les unités D-glucosamine substituées ne forment pas des unités N-acétyl-D-glucosamine après substitution.

Selon une variante, un chitosane substitué présente une substitution des unités D-glucosamine uniquement.

Selon une autre variante, un chitosane substitué présente une substitution des unités D-glucosamine et N-acétyl-D-glucosamine simultanément, et dans lequel le groupe de substitution est lié de manière covalente, selon une variante aux groupes amine du chitosane uniquement, ou selon une autre variante aux groupes amine et hydroxyle du chitosane simultanément.

Selon un mode de réalisation, le degré de substitution des unités D-glucosamine exprimé en nombre de moles d'unités D-glucosamine par rapport au nombre de moles d'unités totales (unités D-glucosamine et N-acétyl-D-glucosamine, substituées ou non) du chitosane substitué, va de 0,1 à 0,5%.

Il a été découvert selon une variante spécifique, qu'un degré de substitution des unités glucosamine, exprimé en nombre de moles d'unités glucosamine sur lesquelles le groupe substituant est attaché par une liaison covalente, par rapport au nombre de moles totales d'unités glucosamine et N-acétyl-glucosamine du chitosane substitué, de 0,1 à 0,5 permettait avantageusement de solubiliser le chitosane substitué, éventuellement en présence d'un agent gélifiant, par exemple un sel de polyol ou de sucre, et plus particulièrement un sel de glycérol phosphate, à un pH et à une température dans une gamme particulièrement adaptée aux applications visées. Il peut être plus simple d'exprimer le degré de substitution par le ratio molaire ou par le ratio massique des réactifs de départ par rapport au chitosane, comme expliqué plus loin.

Selon une variante, le chitosane substitué est soluble de par son degré de substitution dans une solution d'eau tamponnée à pH 7, par exemple avec un tampon phosphate salin (PBS), la solution comprenant une concentration de chitosane substitué de 1% en masse par rapport à la masse de la solution totale, le pH étant mesuré à 8°C.

Selon une variante, notamment lorsque le chitosane substitué présente un caractère zwitterionique, il existe une plage de pH étroite dans laquelle le chitosane substitué est insoluble. Cette particularité est contournée selon l'art antérieur par l'ajout d'un agent gélifiant, par exemple sel de polyol ou de sucre, et plus particulièrement sel de glycérol phosphate, pour que le chitosane substitué soit solubilisé au pH considéré d'intérêt pour l'invention.

En substituant le chitosane, il a été possible de préparer une solution d'un chitosane substitué soluble dans une solution aqueuse dont le pH varie dans une large gamme, alors que le chitosane non substitué n'est soluble qu'à pH en dessous de 5,5 à 6,5. La solution aqueuse de chitosane substitué peut présenter en général un pH allant de 6,5 à 8,5. Cette solution selon l'invention peut être gélifiée. La gélification ou transition sol-gel peut donc être réalisée à pH neutre ou à pH physiologique, comme par exemple à un pH compris entre 7 et 8,5, alors que le chitosane n'est pas soluble à pH 7, le pKa des groupes amine étant d'environ 5,5 (et donc non chargé à pH 7 auquel les groupes amine ne sont pas protonés). Le chitosane substitué présente ainsi une capacité à être solubilisé à différents pH grâce à la présence de groupes substituants qui modifient son profil de solubilisation. De manière surprenante il a été possible de préparer des compositions thermogélifiables avec un chitosane substitué, et en particulier à faible concentration de chitosane, en particulier avec un chitosane de très faible, faible, ou moyenne masse moléculaire, tout en présentant une transition sol-gel réversible ou dont les propriétés du gel résultant sont en adéquation avec les applications envisagées, en particulier comme viscosupplément d'un fluide synovial. Une telle composition comprenant un chitosane substitué présente une transition sol-gel lorsque la température augmente. Ainsi, de manière très avantageuse la transition sol-gel, c'est-à-dire le passage de la solution à l'état fluide à l'état de gel, peut être modulée en fonction du degré de substitution pour que cette transition sol-gel se produise notamment dans des conditions de pH, osmolalité, et température désirées. L'invention permet donc avantageusement, selon une variante, de préparer une composition thermogélifiable fluide à une température inférieure à celle d'utilisation, typiquement à une température inférieure à la température physiologique, par exemple de 37°C, mais qui soit sous forme de gel à la température d'utilisation, typiquement à la température physiologique, par exemple de 37°C, à pH neutre (pH 7) ou à pH physiologique, et par exemple de 7 à 8,5, avec une osmolalité convenable pour l'utilisation envisagée. Il s'agit par exemple d'une osmolalité physiologique.

Selon une autre variante, l'invention concerne une composition thermogélifiable, sous forme de gel avant utilisation, typiquement à température ambiante, et par exemple à température de conservation (par exemple de 4 à 8°C), et à température d'utilisation, typiquement à la température physiologique, par exemple de 37°C, à pH neutre (pH 7) ou à pH physiologique, et par exemple de 7 à 8,5, avec une osmolalité convenable pour l'utilisation envisagée. Il s'agit par exemple d'une osmolalité physiologique.

On entend par « composition thermogélifiable » ou « thermogel » un fluide, et notamment une solution qui présente la propriété de subir une transition sol-gel par modification de la température, de préférence par augmentation de la température, et de préférence encore par augmentation de la température jusqu'à la température physiologique du corps humain ou animal, et ainsi se présenter sous la forme d'un gel à température physiologique après injection ou implantation. La composition thermogélifiable de l'invention peut se présenter sous forme d'hydrogel, c'est-à-dire de gel aqueux.

Selon la présente invention, on entend par gel une composition ne s'écoulant pas sous son propre poids, et plus spécifiquement ne présentant aucun écoulement en l'absence de stimuli extérieur par retournement d'un récipient contenant la composition par exemple pendant 30 secondes, et caractérisée par un module élastique G' supérieur au module de perte G", les modules étant mesurés par rhéologie à l'aide d'un rhéomètre de géométrie « Couette » » ou à l'aide d'un rhéomètre avec une géométrie plan et un balayage en mode oscillatoire de faible amplitude (par exemple rhéomètre « Discovery Hybrid DHR-2 » (TA Instrument)). La différence entre les modules G' et G" du gel sont caractéristiques de la cohésion du gel.

Selon la présente invention, on entend par « fluide » une composition dont s'écoulant sous son propre poids par retournement d'un récipient contenant la composition par exemple pendant 30 secondes et caractérisée par un module élastique G' inférieur au module de perte G", les modules étant mesurés par rhéologie à l'aide d'un rhéomètre à rotation avec cisaillement de Couette (cisaillement du fluide entre deux cylindres coaxiaux).

Avantageusement, il a été découvert que le degré de substitution des unités D-glucosamine permet de moduler la transition sol-gel. Il a notamment été découvert que le degré de substitution des unités D-glucosamine permet de former une composition thermogélifiable sans agent gélifiant, comme par exemple un sel de glycérol. Ainsi l'invention permet avantageusement de fournir une composition thermogélifiable dans laquelle le degré minimal de substitution du chitosane est choisi de manière à ce que le chitosane substitué soit soluble dans la composition thermogélifiable. On peut aussi vérifier la solubilité dans différents tampons. Par « soluble dans l'eau », on entend que le chitosane substitué ne présente pas de trouble visible à l'oeil nu. Plus spécifiquement, on peut confirmer l'absence de trouble par une densité optique inférieure à 0,5, et de préférence inférieure à 0,2, mesurée par spectrométrie UV-visible à la longueur d'onde de 600nm d'un échantillon comprenant de l'eau éventuellement tamponnée par un tampon au pH concerné, par exemple comme l'acétate de sodium à 0,5M, en référence à une cuve de référence ne comprenant que le solvant aqueux utilisé pour l'échantillon mesuré, mais en l'absence du chitosane substitué. Lorsque le chitosane n'est pas suffisamment substitué, la composition n'est pas soluble dans une gamme de pH satisfaisante, par exemple allant de pH=7 à pH=8,5, à température ambiante, et par conséquent n'est pas non plus thermogélifiable lorsqu'on augmente la température.

L'invention permet de fournir une composition thermogélifiable dans laquelle le degré maximal de substitution du chitosane est choisi pour qu'une transition sol-gel s'effectue à une température inférieure à la température d'utilisation, de préférence inférieure à la température physiologique, par exemple à la température de 37°C. La transition sol-gel peut se constater par le croisement des modules G' et G" selon la méthode définie dans l'invention.

Avantageusement, le degré de substitution est déterminé par spectrométrie de résonance magnétique (RMN) du proton en solution en milieu aqueux, à l'aide d'un spectromètre de résonance magnétique, par exemple un spectromètre Bruker de fréquence 400 MHz. Les échantillons sont préparés de la manière suivante : 5 à 6mg de chitosane substitué sont dissout dans 1ml d'eau deutérée. On ajoute 2$\mu$l d'acide chlorhydrique deutéré de concentration 12M à la solution du chitosane substitué afin d'atteindre une zone de pH appropriée pour l'analyse. La zone de pH appropriée dépend de la nature du substituant. Le spectre est enregistré à une température de 70°C, avec un nombre de scans allant de 64 à 256 et un temps de relaxation allant de 1 à 8 secondes. Le spectre obtenu est traité par déconvolution afin de déterminer la valeur de l'intégrale des aires des signaux d'intérêt pour pouvoir calculer le degré de substitution du chitosane substitué.

La méthode de préparation de l'échantillon, les conditions d'enregistrement du spectre RMN, et la formule utilisée pour calculer le degré de substitution doivent être adaptés à chaque type de chitosane substitué, car ils dépendent de la nature et de la position du substituant.

Un exemple de calcul du degré de substitution (DS) est donné ci-dessous pour le cas du chitosane substitué par un groupe succinyle lié au groupe amine des unités D-glucosamine (chitosane succinamide, Formule 1). Les abréviations sont les suivantes : $I_{H2}$ est égal à l'intégrale de l'aire du signal du proton des unités D-glucosamine en position 2 ; $I_{CH2\,succi}$ est égal à l'intégrale de l'aire du signal des protons des deux groupes -CH2 des substituant succinyle liés aux unités D-glucosamine (sur les atomes de carbone en position alpha et beta de la fonction amide) ; $I_{CH3\,acétyle}$ est égal à l'intégrale de l'aire du signal des protons des groupes acétyle des unités N-acétyl-D-glucosamine.

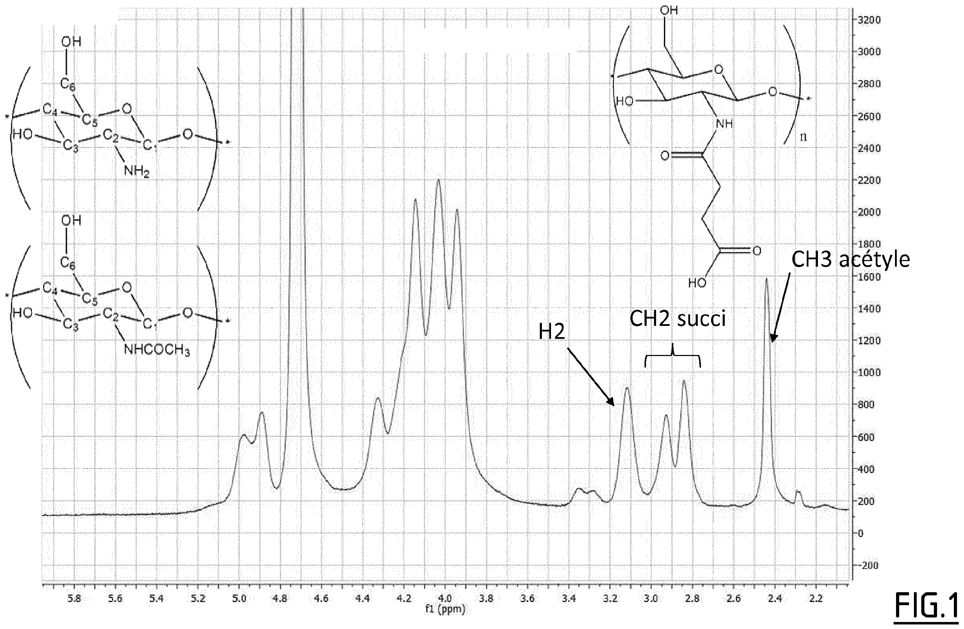

Un exemple de spectre RMN du chitosane succinamide et la formule développée du chitosane succinamide sont illustrés sur la Figure 1.

$$\%DS = \frac{I_{CH2\,succi}/2}{I_{H2} + I_{CH2\,succi}/2 + I_{CH3\,acétyle}/3}$$

**Formule 1-** Calcul du degré de substitution du chitosane substitué par un groupe succinyle par RMN du proton

Si une autre méthode RMN et plus avantageuse pour estimer le degré de substitution de manière fiable, il convient d'utiliser une telle méthode. La formule ci-dessus doit être adaptée par l'homme du métier en ce qui concerne la préparation de l'échantillon et les signaux à intégrer, notamment en fonction de la résolution, de la robustesse et de la position des protons des signaux à utiliser pour le calcul du degré de substitution.

Avantageusement, la composition présente une transition sol-gel thermoréversible.

Avantageusement, la présente invention permet de fournir une composition à faible concentration de chitosane substitué.

Avantageusement, la concentration de chitosane est inférieure à 4%, par exemple inférieure à 3%, ou encore par exemple inférieure à 2% en masse par rapport à la masse totale de la composition (m/m).

Selon une variante spécifique, la concentration de chitosane substitué est inférieure à 1,9% (m/m), exprimée en masse par rapport la masse de la composition finale. Avantageusement, la concentration de chitosane substitué est comprise entre 0,5 et 1,5% (m/m), exprimée en masse par rapport la masse de la composition finale. Selon une variante particulière, la concentration de chitosane substitué est environ de 1,2% (m/m), exprimée en masse par rapport la masse de la composition finale.

Selon une variante particulière, la concentration de chitosane substitué est environ de 2,5% (m/m), exprimée en masse par rapport la masse de la composition finale.

Selon une variante particulière, la concentration de chitosane substitué est environ de 2,0% (m/m), exprimée en masse par rapport la masse de la composition finale.

Selon une variante particulière, la concentration de chitosane substitué est environ de 1,5% (m/m), exprimée en masse par rapport la masse de la composition finale.

Selon une variante particulière, la concentration de chitosane substitué est environ de 1,3% (m/m), exprimée en masse par rapport la masse de la composition finale.

Selon une variante particulière, la concentration de chitosane substitué est environ de 1,1% (m/m), exprimée en masse par rapport la masse de la composition finale.

Selon une variante particulière, la concentration de chitosane substitué est environ de 1,0% (m/m), exprimée en masse par rapport la masse de la composition finale.

Selon une variante particulière, la concentration de chitosane substitué est environ de 0,9% (m/m), exprimée en masse par rapport la masse de la composition finale.

Par ailleurs, le degré de substitution des unités D-glucosamine permet avantageusement de ne pas utiliser une solution à pH acide (typiquement 5,0 à 5,5) pour solubiliser le chitosane puis de devoir nécessairement ajouter un sel de sucre ou de polyol, comme le glycérol phosphate, pour augmenter le pH jusqu'à environ 7. Au contraire, la présente invention permet avantageusement de préparer directement une solution à pH neutre ou à pH physiologique, comme par exemple de 6,2 à 8,5, dans laquelle le chitosane substitué est soluble et présente donc l'avantage également par exemple de n'avoir pas nécessairement besoin d'ajouter une solution acide pour solubiliser le chitosane. Ceci présente l'avantage d'assurer une liberté bien plus importante sur la gamme de pH utilisable pour la composition thermogélifiable, et ainsi préparer des compositions thermogélifiable à pH neutre ou physiologique. On peut par exemple utiliser un agent tampon est un acide ou une base pour ajuster le pH. Par exemple on peut utiliser un tampon basique puis ajuster avec un acide faible le pH de la composition thermogélifiable.

Selon une variante avantageuse, la composition thermogélifiable ne comprend pas d'agent gélifiant. Selon cette variante, la composition thermogélifiable présente la propriété de gélifier par la seule présence du chitosane substitué. En particulier, selon cette variante, la composition ne comprend pas un agent gélifiant tel que défini dans l'invention. En particulier, selon une variante préférée, la composition thermogélifiable de l'invention ne comprend pas de sel de glycérol, et en particulier pas de sel de glycérol phosphate (écrit aussi sous la forme « glycérophosphate »).

Cette variante permet d'éviter la présence de glycérol phosphate ou d'un autre agent gélifiant équivalent dont l'effet technique est uniquement mécanique sur le gel. Selon cette variante, on préfère éviter les composés comme les agents gélifiant qui n'ont pas de bénéfice thérapeutique ni d'interaction de nature biologique/chimique avec le corps du sujet traité.

Selon une variante, la composition comprend un agent gélifiant, de préférence un agent gélifiant induisant une transition sol-gel de la composition, par exemple un sel de glycérol phosphate par exemple sous la forme sodium ou calcium, par exemple sous sa forme pentahydrate, ledit agent gélifiant étant de préférence présent dans la composition à une concentration comprise entre 1 et 20%, de préférence 3 et 9%, en masse par rapport à la masse totale de la composition finale (m/m).

L'agent gélifiant est avantageusement au moins un sel de polyol ou de sucre, y compris l'un quelconque de leurs mélanges.

Parmi les sels de polyol ou de sucre on peut citer notamment les sels de phosphate, et plus particulièrement les sels dibasiques de mono-phosphate de polyol ou de sucre. On peut également citer les sels de sulfate comme par exemple les sels de mono-sulfate de polyol ou de sucre. Parmi les sels de phosphate on peut notamment citer les mono-phosphates dibasiques de glycérol, incluant le glycérol-2-phosphate, le sn-glycérol-3-phosphate et le 1-glycérol-3-phosphate. Selon une variante, il s'agit du beta-glycérol phosphate. Parmi les polyol et sucres pour de tels sels, on peut citer les polyols et sucres suivants: histidinol, acetol, diethylstilbestrol, indoleglycerol, sorbitol, ribitol, xylitol, arabinitol, erythritol, inositol, mannitol, glucitol, palmitoyl-glycerol, linoleoyl-glycerol, oleoyl-glycerol, arachidonoyl-glycerol, fructose, galactose, ribose, glucose, xylose, rhamnulose, sorbose, erythrulose, deoxy-ribose, ketose, mannose, arabinose, fuculose, fructopyranose, ketoglucose, sedoheptulose, trehalose, tagatose, sucrose, allose, threose, xylulose, hexose, methylthioribose or methylthio-deoxy-ribulose, ou l'un quelconque de leurs mélanges.

Selon une variante spécifique la concentration de sel de polyol ou de sucre, et de préférence de glycérol, est comprise entre 1 et 10%. Avantageusement, la concentration de sel de polyol ou de sucre, et de préférence de glycérol, est comprise entre 1 et 7%. Avantageusement, la concentration de sel de polyol ou de sucre est de 2% à 5%. Les valeurs sont exprimées en masse par rapport en masse totale de la composition.

Selon une variante, le sel de glycérol est un glycérol phosphate, et plus précisément un sel de sodium, comme par exemple le disodium glycérol phosphate.

Le sel de polyol ou de sucre, et de préférence le glycérol phosphate, est utilisé pour amener le pH à un pH basique, puis le pH de la composition est ajusté par l'ajout d'un acide, ce qui présente l'avantage de fournir une composition thermogélifiable dont le pH est modulable très facilement et précisément.

Lorsque la composition thermogélifiable ne comprend pas d'agent gélifiant, le pH peut être ajusté par un acide, et par exemple un acide organique faible ou un acide minéral fort (Acide chlorhydrique). Un acide organique faible par exemple utilisé pour ajuster le pH de la composition est l'acide acétique ou l'acide glutamique.

Ainsi, la composition selon l'invention présente un pH supérieur ou égal à 7, par exemple supérieur ou égal à 7,1, et par exemple un pH de 7,2 à 8,5.

Le pH est mesuré sur la composition thermogélifiable finale de sous forme de solution, c'est-à-dire avant la transition sol-gel. Le pH est déterminé en suivant la méthode décrite dans la pharmacopée européenne (EP 2.2.3). Le pH-mètre utilisé est un pH-mètre de la gamme Sartorius muni d'une électrode combiné en verre (PY-P11). Les mesures de pH sont réalisées entre 20 et 25 °C.

Avantageusement, le pH est ajustable dans la gamme large allant de 6,5 à 8,0.

Selon une variante, le pH est supérieur à 7,40.

Selon une variante spécifique, le pH est de 7,50 +/- 0,05.

Selon une autre variante spécifique, le pH est de 7,20 +/- 0,05.

Selon une autre variante spécifique, le pH est de 7,00 +/- 0,05.

Selon une variante préférée, la composition de l'invention présente une transition sol-gel une température supérieure à 30°C, de préférence à une température comprise entre 30 et 50°C, et de préférence entre 32 et 45°C, et encore de préférence entre 35 et 40°C.

Avantageusement, la composition thermogélifiable de l'invention est sous forme fluide à température ambiante, c'est-à-dire entre 20 et 25°C.

Selon une variante préférée, la composition de l'invention est fluide à une température inférieure à 37°C, de préférence à une température inférieure à 35°C, et encore de préférence à une température comprise entre 2°C et 20°C. Selon une variante, la composition de l'invention est fluide à la température de conservation, par exemple de 2 à 8°C, ou à température ambiante étendue, par exemple entre 15 et 30°C.

Selon une variante, la composition thermogélifiable de l'invention est fluide à une température supérieure à 0°C et sous forme de gel à une température supérieure ou égale à 10°C. Selon une autre variante, la composition de l'invention est fluide à une température supérieure à 0°C et sous forme de gel à une température supérieure ou égale à 5°C.

Selon un mode de réalisation, la composition présente une osmolalité de 100 à 700mosm/kg, de préférence de 200 à 650 mosm/kg, par exemple de 200 à 600 mosm/kg, ou encore par exemple de 200 à 550 mosm/kg, de préférence de 200 à 500mosm/kg. L'osmolalité peut être exprimée en mosm/L, mais on parle alors d'osmolarité. Lorsqu'il s'agit d'une composition aqueuse, la densité est proche d'environ 1, l'osmolalité est sensiblement égale à l'osmolarité, comme pour les compositions de l'invention.

Selon une variante, la composition thermogélifiable selon l'invention est iso-osmolaire.

Selon une variante, lorsque la composition de l'invention est destinée à être injectée dans le plasma d'un être humain, il est préférable que l'osmolalité soit comprise entre 250 et 400, et plus spécifiquement entre 280 et 350mosm/kg.

Selon une autre variante, lorsque la composition de l'invention est destinée à être injectée dans le liquide synovial d'un être humain, il est préférable que l'osmolalité soit comprise entre 300 et 490, et de préférence entre 360 et 470mosm/kg.

La détermination de l'osmolalité des solutions est effectuée avec un micro-osmomètre automatique (Osmometer Type 15M de la marque Löser Messtechnik). L'équipement est préalablement calibré avec une solution de 300mosm/kg. L'échantillon est placé dans un récipient prévu à cet effet, et est mis à la température standard de la mesure.

Avantageusement, la composition thermogélifiable de l'invention composition présente à l'état fluide une viscosité apparente dynamique comprise entre 20 et 800mPa.s, par exemple de 40 à 500mPa.s.

La viscosité apparente dynamique est mesurée à l'aide d'un viscosimètre à mobile tournant, par exemple un viscosimètre à mobile tournant de la marque Brookfield, par exemple muni d'une aiguille (« spindle ») de type S18 à une vitesse de 5 tour/min et à une température de 8°C.

Selon une variante, la composition de l'invention présente une viscosité apparente permettant une injection facile dans un dispositif d'injection comme par exemple une seringue, lors de son remplissage. Selon une variante, la composition de l'invention présente une viscosité apparente permettant une injection facile au travers d'une aiguille fine, par exemple une seringue de 22 Gauge, à température ambiante. Par injection « facile », on entend de préférence que la force à exercer sur une telle seringue est inférieure à 50 Newton pour faire écouler la composition thermogélifiable au travers d'une aiguille de 22 Gauge, de préférence une force inférieure à 20 Newton.

La composition thermogélifiable selon l'invention peut être diluée, par exemple dans de l'eau, éventuellement tamponnée. On peut par exemple diluer la composition de l'invention dans un tampon permettant d'ajuster le pH à un pH physiologique. Plus particulièrement on peut par exemple diluer la composition de l'invention dans un tampon acétate (par exemple un tampon acétate de sodium trihydrate 10mM) pour ajuster le pH à environ 7,5.

Pour un gel, le module G' est supérieur à G". Pour une solution, le module G' est inférieur à G". La transition sol-gel est caractérisée par le croisement des modules G' et G".

Lorsque la solution présente un caractère « thermogélifiable », le module G' devient supérieur au module G" au-delà d'une certaine température, et en particulier à une température inférieure à la température physiologique (c'est-à-dire la température à laquelle le produit est injecté ou implanté) ou après un certain temps après implantation ou injection dans le corps à température physiologique.

Le module G' et le module G" se croisent lorsque la température augmente.

Les modules G' et G" sont mesurés par exemple à l'aide d'un rhéomètre à rotation avec cisaillement de Couette qui applique un cisaillement du fluide entre deux cylindres coaxiaux par exemple un rhéomètre ARES de la marque Rheometrics, par exemple avec une fréquence de 1Hz et une déformation de 5%. On peut faire la mesure des modules G' et G" à une certaine température. La mesure des modules donnée ici est réalisée en partant du produit à une certaine température, par exemple la température de stockage du produit de 4°C. On laisse le produit atteindre une certaine température naturellement, par exemple la température physiologique, par exemple 37°C, sans contrôler la vitesse à laquelle la température augmente.

Avantageusement, le module de conservation G' est compris entre 0,001 et 1000, le module de perte G" est compris entre 0,001 et 1000, G' est supérieur à G" à la température physiologique, par exemple 37°C, après gélification.

Selon une variante, avantageusement, le module de conservation G' est compris entre 0,001 et 1000, le module de perte G" est compris entre 0,001 et 1000, G' est inférieur à G" à température de stockage et/ou à température ambiante, et G' est supérieur à G" à la température physiologique, par exemple 37°C, après gélification.

Avantageusement, les modules de conservation G' et de perte G" se croisent lorsque le produit passe d'une température de conservation dans un réfrigérateur, par exemple 2 ou 4°C ou d'une température ambiante, par exemple 18°C à 25°C, à une température physiologique, par exemple 37°C, traduisant la transition sol-gel et le caractère thermogélifiable du système, avec une durée de transition sol-gel adaptée à l'application visée.

La gélification peut se réaliser par maintient à une température suffisante et pendant une durée suffisante pour gélifier la solution de chitosane. Cette gélification est effectuée par exemple dans une étuve par exemple maintenue à 40°C. Selon une variante, la composition thermogélifiable de l'invention est sous forme de gel à une température de 15 °C. Selon la présente invention la gélification peut intervenir *in situ,* c'est-à-dire par exemple après injection dans le corps humain ou animal (à sang chaud). La gélification permet notamment de positionner le gel de manière localisée.

Plus particulièrement, la durée nécessaire à la transition sol-gel est généralement comprise entre 1 seconde et 48 heures après passage de la température de 2 ou 4°C (température de stockage) à 37°C (température physiologique), c'est à dire par exemple *in situ* après injection de la solution dans un corps humain ou animal.

Selon une variante, la transition sol-gel est complète après une durée qui varie entre 5 secondes et 24 heures, de préférence moins de 4 heures, de préférence encore moins de 2 heures après le passage de la température de 2 ou 4°C à 37°C.

Selon une autre variante, la gélification intervient instantanément (gélification intervenue dès l'augmentation de température (et avant la mesure).

Selon une variante, la composition thermogélifiable est sous forme gélifiée. Par exemple la composition de l'invention est conservée dans une seringue sous forme gélifiée.

Selon une variante, il ne faut pas que la composition gélifie dans la seringue d'injection mais gélifie *in situ* à l'emplacement désiré. Il est possible d'effectuer plusieurs cycles de transition sol-gel puisque la composition selon l'invention présente avantageusement une transition sol-gel réversible. Ceci permet avantageusement de stériliser la composition selon l'invention par montée en température, c'est-à-dire typiquement par autoclave. Ceci permet avantageusement de fournir une composition thermogélifiable dont la possibilité d'obtenir la gélification n'est pas affectée par les variations de température, notamment lors du stockage, du transport, ou de la stérilisation.

La composition selon la présente invention étant destinée notamment à être injectée, les propriétés de gélification sont adaptées à son injection via une seringue munie d'une aiguille de taille variable en fonction de l'application visée, par exemple de 19 à 30 Gauge, par exemple de 22 Gauge. Habituellement, la personne réalisant l'injection doit appuyer manuellement sur le piston de la seringue. Il faut donc que la résistance à la compression du fluide non gélifié permette une injection la plus facile possible. Une fois injectée la composition doit gélifier pour présenter les propriétés viscoélastiques requises.

Selon une variante, la force nécessaire à l'écoulement de la composition fluide dans l'orifice d'une aiguille de 22 Gauge est comprise entre 1 et 20 Newton. De préférence cette force est comprise entre 2 et 15 Newton. Selon une variante, cette force est comprise entre 2 et 8 Newton.

Selon une variante, la force nécessaire à l'écoulement de la composition fluide dans l'orifice d'une aiguille de 25 Gauge est comprise entre 2 et 15 Newton. Selon une variante, cette force est comprise entre 2 et 10 Newton.

Selon une variante, la force nécessaire à l'écoulement de la composition fluide dans l'orifice d'une aiguille de 27 Gauge est comprise entre 1 et 20 Newton. De préférence cette force est comprise entre 2 et 15 Newton. Selon une variante, cette force est comprise entre 2 et 10 Newton. L'injectabilité est mesurée à l'aide d'un banc d'essai pour la mesure de propriétés mécaniques, par exemple de la marque Instron Bluehill, muni d'une cellule de force de 500N. Le système d'injection est conçu spécifiquement pour la mesure de la force nécessaire à réaliser l'injection de la solution en poussant le piston de la seringue, munie de l'aiguille avec le diamètre désiré. Le système présente un cylindre métallique d'une hauteur de 15cm pourvu d'une fente verticale de 4cm de large. Le cylindre est surmonté d'une plaque métallique carrée de 10cm de côté. Cette plaque est pourvue d'un trou en son milieu, de 0,5 cm de rayon. La seringue, munie d'une aiguille de diamètre 22 Gauge est mise à la température de 4°C. On applique une vitesse de descente du piston de la seringue constante de 1mm par seconde.

Selon une variante, la composition comprend un tampon, par exemple un tampon acétate ou un tampon phosphate.

Des agents tampon sont connus de l'homme du métier.

De préférence, la composition comprend un sucre réducteur, par exemple le mannitol ou le sorbitol.

Selon une variante, la présente invention concerne une composition thermogélifiable comprenant 0.1 à 5% en masse de chitosane substitué, et de préférence de 1 à 7% de sel de polyol ou de sucre, par exemple de glycérol phosphate, et éventuellement de 0,1 à 10% de sucre réducteur. Avantageusement, la composition de la présente invention comprend de 0,1 à 1,9 % en masse de chitosane substitué et de 2 et 6 % en masse de glycérol, de préférence de glycérol phosphate, et éventuellement de 0,1 à 2,5% en masse de sucre réducteur, par rapport à la masse totale de la composition.

Selon une variante, la présente invention concerne une composition thermogélifiable comprenant 0,1 à 5%, et de préférence 0,5 à 2,5%, en masse de chitosane substitué, éventuellement de 0,1 à 10% de sucre réducteur, et éventuellement de 0,1 à 5%, et de préférence 0,5 à 2,5%, d'acide hyaluronique. Avantageusement, la composition de la présente invention comprend de 0,1 à 2,5% en masse de chitosane substitué, éventuellement de 0,1 à 2,5% en masse de sucre réducteur, et éventuellement de 0,1 à 5%, et de préférence 0,5 à 2,5%, d'acide hyaluronique, par rapport à la masse totale de la composition, ladite composition ne présentant de préférence pas de glycérol ou d'un sel de de glycérol.

Il est avantageux d'utiliser au moins un polyol dans la composition de l'invention (en plus du sel de polyol comme le glycérol phosphate, s'il est présent).

De tels polyols peuvent être par exemple choisis parmi le groupe consistant en : isopropanol, sorbitol, mannitol, alkylène glycol comme le propylène glycol, poly(alkyl glycol), par exemple un poly(éthylène glycol), fructose, galactose, ribose, glucose, xylose, rhamnulose, sorbose, erythrulose, deoxy-ribose, ketose, mannose, arabinose, fuculose, fructopyranose, ketoglucose, sedoheptulose, trehalose, tagatose, sucrose, allose, threose, xylulose, hexose, methylthio-ribose, methylthio-deoxy-ribulose, et l'un quelconque de leurs mélanges.

Selon une variante spécifique, le réactif qui permet la substitution est l'anhydride succinique. Selon cette variante spécifique, le chitosane substitué est un chitosane succinamide, c'est-à-dire un chitosane avec des groupes « succinyle » liés de manière covalente à l'atome d'azote des groupes amine des unités D-glucosamine.

Le chitosane est modifié par substitution selon une réaction impliquant un chitosane et un agent de substitution dans lequel le chitosane et l'agent de substitution sont mis à y réagir pour obtenir le chitosane substitué sur les groupements D-glucosamine.

L'invention concerne également un procédé de préparation de chitosane substitué.

Ce procédé comprend notamment :

- une étape de dissolution du chitosane dans une solution aqueuse, de préférence de l'eau, de préférence en ajustant le pH à un pH acide auquel le chitosane est soluble;
- une étape de réaction de substitution du chitosane avec un agent de substitution ;
- une étape d'arrêt de la réaction de substitution de préférence à un degré de substitution du chitosane allant de 10 à 50%, exprimé en nombre de mole du substituant par rapport au nombre de mole d'unités totales, par exemple par modification du pH du milieu réactionnel, ou par précipitation du chitosane dans un « non-solvant » dans lequel le chitosane substitué n'est pas soluble ;
- la purification du chitosane substitué.

On entend par « non-solvant » un solvant dans lequel le chitosane situé n'est pas soluble, c'est-à-dire qu'un trouble est observable à l'oeil nu. De tels solvants sont par exemple des solvants polaires comme par exemple l'éthanol, méthanol, l'acétone, etc.

L'étape de dissolution du chitosane dans une solution aqueuse peut être effectuée dans l'eau en ajoutant un acide approprié.

Lors de l'étape de réaction, la quantité d'agent de substitution peut-être adaptée en fonction du degré de substitution souhaité pour le chitosane substitué. Lors de l'étape de réaction, la durée de réaction peut-être adaptée en fonction du degré de substitution souhaité pour le chitosane substitué. Avantageusement, la réaction est arrêtée pour mettre fin à la réaction de substitution du chitosane selon degré de substitution souhaité pour le chitosane substitué.

Différentes réactions sont connues pour substituer un chitosane. On peut se référer par exemple à la demande WO 2010/142507 notamment relative à la préparation de dérivés de chitosane.

Le chitosane substitué peut comprendre des unités D-glucosamine et N-acétyl-glucosamine, dans lesquelles au moins certaines des unités sont greffées (ou couplées) par un ou plusieurs groupes fonctionnels qui peuvent être identiques ou différents.

Selon une variante, le chitosane substitué comprend des unités glucosamine et N-acétyl-glucosamine, lesdites unités comprenant un ou plusieurs groupes fonctionnels. Des modifications chimiques sont par exemple réalisées par couplage d'une ou plusieurs fonctions des unités N-acétyl-glucosamine, c'est-à-dire les fonctions hydroxy et/ou des fonctions amine, et/ou des unités glucosamine, c'est-à-dire des fonctions hydroxy et/ou des fonctions amine.

Selon un mode de réalisation particulier, les groupes fonctionnels présents sur le chitosane substitué sont choisis parmi le groupe comprenant, sans y être limité les groupes fonctionnels hydrophobes, les groupes fonctionnels hydrophiles, les groupes fonctionnels ioniques, et l'une quelconque de leurs combinaisons. Selon une variante particulière, les groupes fonctionnels hydrophobes sont choisis parmi le groupe consistant en les groupes alkyles, alcényles, araalkyles, alkaryles, et l'une quelconque de leurs combinaisons. Ces groupes comprennent en général entre 1 et 100 atomes de carbone, et plus généralement 1 entre 50 atomes de carbone, par exemple entre 1 et 25, ou encore 1 et 10 atomes de carbone, un ou plusieurs des atomes de carbone pouvant être remplacés par exemple par un hétéroatome et/ou un atome choisis parmi le bore, l'azote, l'oxygène, le soufre, le silicium, le germanium, une fonction ester, amide, urée, uréthane, et l'une quelconque de leurs combinaisons, et dans lesquels un ou plusieurs atomes d'hydrogène peuvent être remplacés par exemple par un hétéroatome et/ou un atome choisi parmi le groupe consistant en les atomes d'halogène, les groupes alcoxy, amide, et l'une quelconque de leurs combinaisons.

Selon un autre mode de réalisation, les groupes fonctionnels hydrophobes sont choisis parmi le groupe consistant en les acides carboxyliques, les acides organosulfoniques, les polyéthers, les polyethers d'amines, les polyesters, les stérols, les porphyrines et l'une de leurs combinaisons.

Selon une autre variante, les groupes fonctionnels hydrophiles sont choisis parmi le groupe consistant en les diamines, polyamines, diols, polyols, diacides, polyacides, éthers couronnes, les glymes, les polyalcényléthers, les polyalcénylamines, polyalcénylétheramines, les acides polyacrylique, les polyvinylacools, et l'une quelconque de leurs combinaisons.

Selon une autre variante particulière, les groupes fonctionnels ioniques sont choisis parmi le groupe consistant en les sels métalliques, les sels d'ammonium, les sels de phosphonium, les sels de sulfate, les sels d'acides carboxyliques, les sels de phosphate, les diacides carboxyliques, les polyacides carboxyliques, une fonction acide carboxyliques étant utilisée pour former une liaison covalente avec le chitosane, les diamines, les polyamines, une fonction amine étant utilisée pour former une liaison covalente avec le chitosane, et l'une quelconque de leurs combinaisons.

Selon une variante particulière, le groupe fonctionnel substituant le chitosane, est un groupe aminoalkyle. Par exemple le groupe fonctionnel substituant le chitosane est un groupe aminoéthyle.

Selon une variante, le chitosane substitué est :

- un chitosane amino-alkyl, tels que par exemple un chitosane amino-éthyle, etc. et leurs équivalents ;
- un chitosane N-alkyl ou O-alkyl hydrophobisé, tel que par exemple un chitosane éthanoyl, propanoyol, butanoyl, pentanoyl, hexanoyl, heptanoyl, octanoyl, nonaoyl, decanoyl, ou dodecanoyl et leurs équivalents ;
- un chitosane chargés positivement, tels que par exemple un chitosane trialkyl ammonium (par ex. un triméthyl-chitosane ou TMC) et leurs équivalents ;
- un chitosane N-(2-hydroxy)propyl-3-triméthylammonium et son contre-ion comme un chlorure et leurs équivalents ;
- un chitosane chargé négativement, tel qu'un chitosane succinamide (succinyl chitosan), un chitosane N,O-carboxyalkyl, un chitosane N,O-sulfoalkyl, et leurs équivalents ;
- un chitosane neutre tels que par exemple le chitosane N,O-acétyl, un chitosane N,O-alkyl, éthanoyl, propanoyl, butanoyl, pentanoyl, hexanoyl, heptanoyl, octanoyl, nonaoyl, decanoyl, dodecanoyl, et leurs équivalents ;
- un chitosane zwitterionique, tel que par exemple un chitosane comprenant des polymères hydrophobes, tel que par exemple un polyester aliphatique, tels que les homopolymères et copolymères d'acide lactique, d'acide glycolique, d'epsilon-caprolactone, de p-dioxanone de polyesters aliphatiques et/ou aromatiques; de polyamides aliphatiques; polymères éthyléniques et leurs copolymères; les polymères et copolymères de propylène; les polycarbonates; les polyacrylates, et leurs équivalents.

Selon un mode de réalisation particulier, d'autres dérivés peuvent être utilisés dans le cadre de la présente invention et comprennent les chitosanes O-substitués dont la substitution est réalisée sur les groupes hydroxyles des unités glucosamines et/ou N-acétyl-glucosamine.

Selon une variante, le chitosane présente comme substituant des polymères hydrophobes, choisi de préférence parmi le groupe consistant les polyesters aliphatiques, et polyamides aliphatiques, les homopolymères ou les copolymères d'alcène, tels que par exemple les polymères d'éthylène ou de propylène, les polycarbonates, les polyacrylates, et l'une quelconque de leurs combinaisons.

Selon une variante, le chitosane présente comme substituant un polyester aliphatique, et en particulier un polylactide.

Comme méthode de substitution du chitosane on peut également citer la réaction décrite dans le brevet américain US 7 838 643, et les chitosanes en résultant.

On peut également citer le brevet US 3 953 608.

Selon une variante, on peut utiliser comme agent de substitution par exemple un agent N-alkylant, ou un anhydride d'alkyle, comprenant éventuellement une ou plusieurs insaturations, comprenant éventuellement un ou plusieurs hétéroatomes et/ou fonctions (comme par exemple éther, thiéther, carboxy, ester, amide, etc) comprenant éventuellement un ou plusieurs substituants (comme alkyl, amine, hydroxyl, carboxyl, acide carboxylique, etc).

Selon une variante, on peut citer à titre d'exemples l'anhydride succinique, l'anhydride glutamique, l'anhydride acétoxy succinique, l'anhydride méthylsuccinique, l'anhydride diacétyl tartrique, l'anhydride diglycolique, l'anhydride maléique, l'anhydride itaconique, l'anhydride citraconique, et l'un quelconque de leurs mélanges.

En théorie, tout agent de substitution permettant de fournir un chitosane soluble dans la composition finale, à pH neutre ou physiologique conviendrait.

Lorsque l'étape d'arrêt de la réaction est réalisée par précipitation, la purification peut consister en la séparation du chitosane substitué insoluble et du non-solvant.

Après purification, le procédé de l'invention peut comprendre une étape de séchage du chitosane substitué, puis éventuellement de broyage de celui-ci pour obtenir une poudre.

Selon une variante spécifique, l'agent de substitution est l'anhydride succinique. Le chitosane substitué est un chitosane succinamide.

Le chitosane succinamide peut être obtenu de la manière suivante :

- Dissolution du chitosane dans une solution aqueuse à pH inférieur à 6,5 ;
- La solution est maintenue à une température comprise entre 0°C et 100°C de préférence entre 20°C et 50°C et encore de préférence entre 25°C et 35°C.
- Après dissolution du chitosane, ajout de l'anhydride succinique, par exemple sous forme poudre, à la solution de chitosane. La quantité d'anhydride succinique et le nombre d'ajout d'anhydride succinique son adaptés en fonction du degré de substitution du chitosane substitué désiré.
- Après un temps suffisant pour qu'un degré de substitution minimal soit atteint, par exemple après une durée minimale de 15 min, la réaction est stoppée. La réaction peut être stoppée par exemple par modification du pH du milieu réactionnel ou par une étape de précipitation dans un non-solvant tel que l'éthanol, le méthanol, l'acétone,...
- Le chitosane substitué est ensuite avantageusement purifié, par exemple par une technique de dialyse, ou par des cycles de précipitation/solubilisation dans un non-solvant, ou par une technique de filtration tangentielle.

- Le produit purifié est ensuite de préférence séché. On peut sécher le produit par exemple par spray-drying (atomisation), ou dans une étuve (sous vide ou à pression atmosphérique), ou encore par lyophilisation..

Le pH de la solution aqueuse de chitosane peut être ajusté par exemple avec une solution d'acide faible, tel que l'acide acétique, l'acide lactique, etc., pour dissoudre le chitosane.

Le degré de substitution du chitosane peut varier avantageusement de 10 à 30%, de préférence de 15 à 30%, et encore de préférence de 15 à 25%, exprimé en nombre de moles du substituant par rapport au nombre de moles d'unités totales. En particulier, la présente invention concerne une composition thermogélifiable comprenant un chitosane substituée présentant un degré de substitution de 10 à 30%, de préférence de 15 à 30%, et encore de préférence de 15 à 25%, ladite composition ne comprenant pas de glycérol phosphate.

Le degré de substitution du chitosane succinamide est corrélé au ratio massique des réactifs par rapport au chitosane au départ de la réaction. Selon une variante, on préfère utiliser un ratio massique des réactifs supérieur à 0,13 dans le cas d'un chitosane succinamide. Selon une variante, on préfère utiliser un ratio massique des réactifs inférieur à 0,2 dans le cas d'un chitosane succinamide.

Selon une variante spécifique, l'agent de substitution est un agent alkylant des groupes amines des unités D-glucosamine. Le chitosane substitué est avantageusement un chitosane alkylé sur les unités D-glucosamine. Comme agents alkylants, on peut citer les halogénoalkyles comme les halogénométhyles, comme l'iodure de méthyle, le bromure de méthyle, les chlorure d'acyles, comme par exemple ceux portant un ou ou plusieurs groupe carboxyméthyle, aminoéthyle et/ou triméthyle, etc.

Selon cette variante, le chitosane substitué est un chitosane N-alkylé. Selon une variante spécifique, le chitosane substitué est le chitosane portant un groupe de substitution tri-méthyle (N,N,N-trimethyl chitosan, en abrégé « TMC »).

Selon une variante, le chitosane alkylé, de préférence le TMC, est obtenu avec un ratio molaire des réactifs allant de 0,1 à 0,35, et de préférence de 0,15 à 0,30, exprimé nombre de moles d'agent alkylant par rapport au nombre de moles de groupes amine initialement présents dans le chitosane.

Le chitosane est par exemple référencé sous le numéro CAS 9012-76-4.

Le chitosane utilisé pour l'invention est avantageusement d'origine fongique, et de préférence issu du mycélium d'un champignon du type *Ascomycète,* et en particulier d'*Aspergillus niger*, et/ou d'un champignon *Basidiomycète,* et en particulier *Lentinula edodes* (shiitake) et/ou *Agaricus bisporus* (champignon de Paris). De préférence, le chitosane est issu d'*Agaricus bisporus.* Le chitosane est de préférence très pur, c'est-à-dire contenant peu d'impuretés issues de son origine fongique, et d'une qualité microbiologique compatible avec son utilisation comme implant ou composition pharmaceutique. Une méthode de préparation du chitosane est celle décrite dans les brevets WO 03/068824 (EP 1483299 ; US 7 556 946).

Le chitosane préparé peut-être de différentes masses moléculaires, et généralement allant de 10 000 à 300 000.

Selon une variante, la masse moléculaire moyenne est comprise entre 20 000 et 60 000.

Selon une autre variante, la masse moléculaire moyenne est comprise entre 60 000 et 100 000.

Selon une autre variante, la masse moléculaire moyenne comprise entre 100 000 et 120 000.

Selon une autre variante, la masse moléculaire moyenne comprise entre 120 000 et 150 000.

Selon une autre variante, la masse moléculaire moyenne est comprise entre 150 000 et 220 000.

Il est possible d'hydrolyser le chitosane afin de diminuer sa masse moléculaire.

De préférence ici, la masse moléculaire moyenne est la masse moléculaire moyenne en viscosité (Mv), calculée à partir de la viscosité intrinsèque selon l'équation de Mark-Houwink. La viscosité intrinsèque est mesurée par viscosimètrie capillaire, avec un viscosimètre capillaire de type Ubbelohde, selon la méthode de la monographie de la Pharmacopée Européenne EP2.2.9. On mesure le temps d'écoulement de la solution à travers un tube capillaire adapté (Lauda, par exemple le tube capilaire Ubbelohde 510 01 de diamètre 0.53mm) à l'aide d'un viscosimètre automatique Lauda Visc, d'abord à la concentration initiale en chitosane, puis pour plusieurs dilutions, par exemple selon les recommandations de la méthode EP2.2.9. On en déduit la viscosité intrinsèque réduite pour chacune des concentrations. On porte la viscosité réduite en fonction de la température, et on extrapole la valeur à la concentration 0 pour en déduire la viscosité intrinsèque. Il faut par exemple porter la viscosité réduite ($\eta_{réd}$ en ml/g) des i dilutions en fonction de la concentration C des i dilutions (g/ml) selon la formule 5.

$$\text{Formule 2.} \quad [\eta_{réd}] = (t_1 - t_0) - (1 - C).$$

POur calculer la masse viscosimétrique moyenne, on applique l'équation de Mark-Houwink avec les constantes k et alpha recommandées par Rinaudo et al. (Int. J. Biol. Macromol, 1993, 15, 281-285), selon le DA du chitosane, selon l'une des trois formules suivantes.

Formule 3. Mv = ([η]/0,082)$^{(1/0,76)}$, pour un DA de 2% ;
Formule 4. Mv = ([η]/0,076)$^{(1/0,76)}$ pour un DA de 10% (par exemple 11.5%) ;
Formule 5. Mv = ([η]/0,074)$^{(1/0,76)}$, pour un DA de 20% (par exemple 21%).

Pour les valeurs de DA intermédiaires, on réalise une interpolation linéraire pour calculer la masse viscosimétrique moyenne (Mv).

De préférence, le chitosane utilisé est d'une masse moléculaire moyenne comprise entre 120 000 et 150 000 ou encore comprise entre 150 000 et 220 000.

Selon une variante spécifique, le chitosane substituée présente, de préférence une masse moléculaire moyenne de 150 000 à 220 000 et un degré de substitution allant de 10 à 50 %, la masse moléculaire étant de préférence exprimée avant substitution.

Selon une autre variante spécifique, le chitosane substituée présente, une masse moléculaire moyenne de 120 000 à 150 000 et un degré de substitution allant de 12 à 40%, de préférence de 12 à 30%, voire de 20 à 30% la masse moléculaire étant de préférence exprimée avant substitution.

Avantageusement, le chitosane présente un degré d'acétylation compris entre 5 et 50%. Le degré d'acétylation est exprimé en nombre d'unités N-acétyl-D-glucosamine par rapport au nombre d'unités totales N-acétyl-D-glucosamine et D-glucosamine présentes. Selon une variante, le degré d'acétylation est compris entre 20 et 45%.

Selon une variante, le degré d'acétylation est compris entre 5 et 20%.

Selon une variante, le degré d'acétylation est compris entre 15 et 25%.

Selon une variante, le degré d'acétylation est compris entre 20 et 30%.

Selon une variante, le degré d'acétylation est compris entre 25 et 40%.

Le degré d'acétylation est déterminé par potentiométrie. Le chitosane est dissout dans une solution d'acide chlorhydrique. L'excès d'acide chlorhydrique n'ayant pas réagi avec les fonctions amines du chitosane est dosé par une solution titrée d'hydroxyde de sodium. On en déduit ainsi le nombre de mole d'unité D-glucosamine présentes dans le chitosane et donc par soustraction le degré d'acétylation.

Le chitosane présente avantageusement un degré d'acétylation contrôlé. Par les termes « chitosane ayant un degré d'acétylation contrôlé » on entend un produit dont le degré d'acétylation, c'est-à-dire la proportion des unités N-acétyl-glucosamine, peut être ajustée de manière contrôlée.

Avantageusement, la composition de l'invention peut comprendre également un autre biopolymère que le chitosane substitué. Selon une variante avantageuse, le biopolymère est un polysaccharide, par exemple l'acide hyaluronique ou le hyaluronate de sodium.

L'acide hyaluronique peut présenter une masse moléculaire jusqu'à 4MDa. La masse moléculaire de l'acide hyaluronique peut reflétée par sa viscosité intrinsèque. L'acide hyaluronique peut présenter une viscosité intrinsèque allant de 1 à 4 m3/kg, et par exemple être caractérisé comme de faible (par exemple environ 1 à 2m$^3$/kg) ou haute (par exemple environ 2 à 4m$^3$/kg) masse moléculaire.

Avantageusement, la concentration d'acide hyaluronique est inférieure à 4%, par exemple inférieure à 3%, ou encore par exemple inférieure à 2% en masse par rapport à la masse totale de la composition (m/m).

Selon une variante spécifique, la concentration d'acide hyaluronique est inférieure à 1,9% (m/m), exprimée en masse par rapport la masse de la composition finale. Avantageusement, la concentration d'acide hyaluronique est comprise entre 0,5 et 1,5% (m/m), exprimée en masse par rapport la masse de la composition finale. Selon une variante particulière, la concentration d'acide hyaluronique est environ de 0,9%, de 1,0%, de 1,1%, de 1,2%, de 1,3%, de 1,5% (m/m), exprimée en masse par rapport la masse de la composition finale.

Le ratio entre le chitosane et l'acide hyaluronique peut par exemple varier de 5 à 95 %, par exemple de 10 à 90%, et encore par exemple de 30 à 70% de chitosane substitué et de 5 à 95 %, par exemple de 10 à 90%, et encore par exemple de 30 à 70% respectivement, d'acide hyaluronique, les pourcentages exprimés par rapport : masse sèche de chitosane substitué /masse sèche d'acide hyaluronique). Selon une variante, ce ratio entre le chitosane et l'acide hyaluronique est de 1/1 (soit 50% de chitosane et 50% d'acide hyaluronique). Selon une autre variante, le ratio d entre le chitosane et l'acide hyaluronique est de 1,5/0,5 (soit 75% de chitosane et 25% d'acide hyaluronique).

Avantageusement, la composition thermogélifiable à base de chitosane substitué, en présence d'acide hyaluronique, forme un gel. Avantageusement, selon cette variante, les propriétés rhéologiques de la composition thermogélifiable sont modulables avec la concentration, le rapport entre le chitosane et d'acide hyaluronique, et la masse moléculaire d'acide hyaluronique.

Avantageusement les propriétés du gel sont conservées en présence d'acide hyaluronique.

Le gel comprenant le chitosane substitué, éventuellement en mélange avec l'acide hyaluronique, permet avantageusement de présenter une bonne cohésion et une bonne capacité d'absorption des chocs du gel à faible fréquence d'oscillation, par exemple à une fréquence similaire à celle imposée sur le liquide synovial d'un genou durant la marche (représentée par la valeur du module G' à 0,6 Hz) et une bonne capacité de lubrification d'une articulation (représentée par la viscosité au repos).

L'invention concerne également un procédé de préparation d'une composition thermogélifiable selon l'invention.

Selon une variante, le procédé comprend typiquement :

- la dissolution d'un chitosane substitué dans une solution aqueuse, de préférence de l'eau, éventuellement tamponnée, de préférence à pH compris entre 6,2 et 8,5 et de préférence entre 6,5 et 7,5 ;
- l'ajustement éventuel du pH à un pH physiologique, par exemple par ajout d'un agent tamponnant ;
- l'ajustement éventuel de l'osmolalité de la composition ;
- l'ajustement éventuel de la viscosité de la composition.

Selon une variante, le procédé comprend typiquement :

- la dissolution d'un chitosane substitué dans une solution aqueuse, de préférence de l'eau, éventuellement tamponnée, de préférence à pH compris entre 6,2 et 8,5 et de préférence entre 6,5 et 7,5 ;
- l'ajustement éventuel du pH à un pH physiologique ;
- le mélange avec une solution de sel de sucre ou de polyol, comme par exemple une solution de sel de glycérol phosphate ;
- l'ajustement éventuel du pH à un pH physiologique, par exemple par ajout d'un agent tamponnant ;
- l'ajustement éventuel de l'osmolalité de la composition ;
- l'ajustement éventuel de la viscosité de la composition.

Le procédé peut comprendre l'ajout d'autres composants de la composition, le cas échéant.

Selon une variante la dissolution du chitosane s'effectue dans de l'eau comprenant un sucre réducteur, comme par exemple le mannitol.

Selon une variante, l'agent tamponnant est un tampon acétate.

Selon une variante, l'agent tamponnant est un tampon phosphate.

Avantageusement, le procédé comprend également une étape ultérieure de remplissage d'un dispositif d'injection, comme par exemple une seringue, avec la composition selon l'invention. Avantageusement, le dispositif d'injection, comme par exemple une seringue peut ensuite subir une stérilisation à la vapeur. Ce dispositif, par exemple une seringue, peut ensuite être emballée, de préférence de manière stérile.

Il est avantageux d'utiliser un chitosane présentant un degré de pureté suffisant pour l'application envisagée.

La présente invention concerne plus particulièrement une composition injectable comprenant ou consistant en une composition thermogélifiable selon l'invention.

Selon une variante, la composition selon l'invention est utilisée comme composition pharmaceutique injectable ou dispositif médical injectable ou implantable.

La présente invention concerne plus particulièrement une composition selon l'invention pour une utilisation pour un traitement thérapeutique, par exemple comprenant l'injection par voie sous-cutanée, intradermique, intraoculaire, ou intra-articulaire, de ladite composition, par exemple pour la réparation ou le comblement d'au moins un tissu corporel nécessitant une réparation ou un comblement.

Selon une variante, le tissu corporel est choisi parmi les tissus appartenant aux cordes vocales, muscles, ligaments, tendons, cartilages, organes sexuels, os, articulations, yeux, derme, épiderme, une ou plusieurs couches de la peau, le mésoderme, ou l'une quelconque de leurs combinaisons, et plus particulièrement aux joints articulaires.

La présente invention concerne plus particulièrement une composition selon l'invention pour le traitement d'une arthrose, ou la réparation d'un défaut de cartilage, par exemple par injection dans le fluide synovial ou après mélange avec le sang et implantation dans le cartilage.

La présente invention concerne plus particulièrement un dispositif médical, par exemple implant médical, caractérisé en ce qu'il comprend ou consiste en une composition selon l'invention.

La présente invention peut également comprendre un ou plusieurs additifs ou excipients permettant de moduler ses propriétés. Selon une variante spécifique la composition de l'invention comprend une suspension de particules, par exemple des particules solides ou de gel.

La présente invention concerne également une composition selon l'invention pour une utilisation dans une méthode de traitement ou de soin esthétique par comblement du derme (« dermal filling »). Il s'agit notamment par exemple d'injecter une composition selon l'invention en sous-cutané ou en intra-dermique.

La présente invention concerne également une composition selon l'invention pour une utilisation dans une méthode de traitement superficiel de la peau par injection multiple par voie intra-dermique. De telles compositions peuvent être typiquement utilisées en dermatologie, comme traitements à visée esthétique. Une telle méthode a par exemple pour but de regonfler la peau pour lui faire perdre un aspect fripé (traitement des rides et/ou ridules). Un tel traitement peut être adressé à un sujet souhaitant donner un aspect rajeuni à sa peau.

La présente invention concerne également une composition selon l'invention pour une utilisation dans une méthode de traitement ou de soin esthétique dans laquelle la composition est un agent viscosupplément. Il s'agit ici par exemple d'injecter au niveau intra-articulaire la composition de l'invention notamment pour limiter les frottements de la membrane synoviale située de part et d'autre de l'articulation.

La présente invention concerne également une composition selon l'invention pour une utilisation comme vecteur cellulaire, d'un ou plusieurs type cellulaire, et/ou un ou plusieurs d'agents actifs. Il peut s'agir d'agents actifs d'un point de vue pharmaceutique ou biologique. La composition de l'invention peut en effet être compatible avec la présence de cellules, de préférence de cellules vivantes. Parmi les cellules vivantes d'intérêt, on peut citer par exemple : chondrocytes (cartilage articulaire), fibrochondrocytes (ménisque), fibroblastes de ligament (ligament), fibroblastes de peau (peau), ténocytes (tendons), myofibroblastes (muscle), les cellules souches mésenchymales, les globules rouges (sang) et kératinocytes (peau). La composition de l'invention peut également être visée comme vecteur thérapeutique pour la délivrance ciblée et/ou à libération contrôlée d'au moins un agent thérapeutique.

Selon une variante, on ajoute du sang, ou du plasma, ou un lysat plaquettaire, ou du plasma riche en plaquettes, ou tout fluide biologique avec la composition de l'invention permettant par exemple d'augmenter les performances du produit.

Selon une variante, la composition selon l'invention est formulée sous une forme solide (par exemple un film ou une mousse poreuse), qui se solubilise une fois implanté.

Selon une variante, la composition est formulée sous une forme d'une composition nébulisable (spray).

La présente invention concerne également une composition selon l'invention pour une utilisation dans une méthode de traitement ou de soin esthétique d'un ou plusieurs tissus ou organes affectés par une température excessive, comme dans le cas d'une brûlure.

La présente invention concerne également une composition à titre de fluide synovial artificiel comprenant ou consistant en la composition thermogélifiable selon l'invention.

La composition selon la présente invention permet de mimer un fluide synovial sain ou améliorer un fluide synovial sain ou défectueux en cherchant à améliorer ses propriétés d'absorption des chocs (identifiable par le module d'élasticité G'), tout en étant facilement injectable pour remplir une seringue par exemple ou être injectée dans le corps humain ou animal. Pour indication, le module élastique G' du liquide synovial sain est compris entre 40 et 100Pa, et son module de perte G" est compris entre 1 et 10Pa.

Selon une variante, la composition thermogélifiable est injectée sous forme fluide, puis gélifie *in situ.*

Selon une variante, la composition thermogélifiable est injectée sous forme de gel. Avantageusement, selon cette variante, le gel est facilement injectable au travers d'une aiguille fine.

En général les gammes de valeurs d'osmolalité et pH recherchées dans les applications biomédicales sont proches des valeurs suivantes :

-Osmolalité :

- Iso-osmolaire au plasma : 285-295mosm/kg ;

- Iso-osmolaire au liquide synovial : 404 +/- 57 mosml/kg, d'après « Clin Orthop Relat Res, 1988, 235, 289-95 » et « Biochem Biophys Res Comm, 2012, 422, 455-461 » ;

-pH :

Un pH physiologique est en général au-dessus de 6,8, en particulier au-dessus de 7,0, et en particulier de 7,4 (comme pour le plasma ou un liquide synovial).

Le pH du plasma est en général de 7,4. Le pH du liquide synovial est en général de 7,768 +/- 0,044 selon « J Bone Joint Surg Br, 1959, 41-B(2), 388-400 » ; ou de 7,3 selon « Acta Orthop Scand, 1969, 40, 634-641 », ou encore d'après « Clin Rheumatol 2006, 25, 886-888 ».

Le pH synovial dans les cas d'ostéoarthrite est considéré comme étant en général plus faible que celui du fluide synovial sain.

De manière tout à fait surprenante, il a été découvert qu'une composition thermogélifiable selon la présente invention présente de très bonnes propriétés en mélange avec un fluide synovial, en particulier un fluide synovial issu du genou d'un être humain atteint d'ostéo-arthrose.

Contrairement à l'effet qui est observé avec des compositions de l'art antérieur à base d'acide hyaluronique, dont les propriétés de viscosupplément chutent lorsque l'acide hyaluronique est dilué avec le fluide synovial d'un patient atteint d'ostéo-arthrose, des compositions thermogélifiable de l'invention, en mélange avec un fluide synovial conservent leurs propriétés de gel, même après cette dilution dans le fluide synovial. De manière encore plus surprenante, les compositions thermogélifiable selon l'invention présentent des propriétés rhéologiques renforcées par mélange avec un fluide synovial, en particulier un fluide synovial issu du genou d'un être humain atteint d'ostéo-arthrose.

On peut parler d'effet de synergie entre la composition thermogélifiable de l'invention et un fluide synovial.

Ainsi, la présente invention concerne un mélange d'un fluide synovial avec une composition thermogélifiable telle que définie par la présente invention, par exemple selon un ratio volumique composition thermogélifiable/fluide synovial allant de 20/80 à 80/20 (v/v), et par exemple de 50/50 (v/v).

Avantageusement, la composition selon la présente invention est stérile. Très avantageusement, la composition selon la présente invention est stérilisée par montée en température, de préférence sous autoclave.

Selon une variante, les compositions de l'invention sont transparentes ou translucides.

Par « translucide » on entend que l'on peut distinguer un objet lorsqu'on place sa composition entre l'oeil de l'observateur et l'objet. Par « transparente » on entend que l'on peut distinguer des caractères alphanumériques lorsqu'on place la composition entre l'oeil de l'observateur et les caractères observés. En général on réalise cette évaluation avec une épaisseur composition d'environ 1cm.

Selon une variante, la composition de l'invention est incolore, c'est-à-dire en particulier qu'un observateur à l'oeil nu n'attribue pas de couleur spécifique à la composition.

L'invention concerne en particulier des articles ou packaging, de préférence stériles, comprenant un ou plusieurs dispositifs d'injection pré-remplis d'une composition thermogélifiable selon l'invention. Il s'agit typiquement de seringues pré-remplies.

La composition de l'invention peut avantageusement être stérilisée. Selon une variante, la composition de l'invention est stérilisée à la vapeur, par exemple par une élévation de la température à une température supérieure à 100°C, et de préférence supérieure à 120°C, par exemple entre 121 et 138°C, sous autoclave, pendant une durée suffisante à la stérilisation, par exemple en général de 15 à 20 minutes.

L'invention couvre en particulier une composition thermogélifiable stérilisée comprenant un chitosane substitué présentant des unités N-acétyl-D-glucosamine, des unités D-glucosamine, et des unités D-glucosamine substituées autres que les unités N-acétyl-D-glucosamine et éventuellement des unités N-acétyl-D-glucosamine substituées.

Avantageusement, la composition thermogélifiable stérilisée présente une transition sol-gel réversible.

Selon une variante, la composition est stérilisée à la vapeur.

Selon une variante, la composition est stérilisée par augmentation de la température, de préférence sous autoclave.

Avantageusement, selon une variante, la composition thermogélifiable stérilisée n'est pas gélifiée après stérilisation.

Avantageusement, selon une variante, la composition thermogélifiable stérilisée est gélifiée à température supérieure à 10 ou 15°C, avant et après stérilisation.

Selon une variante, la solution étant constituée de ou comprenant une composition thermogélifiable selon l'invention.

Selon une variante la composition est stérilisée à la vapeur avec une élévation de la température à une température supérieure à 100°C, de préférence sous une enceinte fermée. Avantageusement, elle est stérilisée par une élévation de la température à une température supérieure à 120°C sous autoclave.

L'invention concerne selon un autre aspect un procédé de stérilisation d'une telle composition.

Plus particulièrement l'invention concerne un procédé de stérilisation d'une composition thermogélifiable, caractérisé en ce qu'il comprend au moins les étapes suivantes :

- placer une composition thermogélifiable comprenant un chitosane substitué présentant des unités N-acétyl-D-glucosamine, des unités D-glucosamine, et des unités D-glucosamine substituées autres que les unités N-acétyl-D-glucosamine dans l'enceinte d'un autoclave ;
- élever et maintenir la température de l'enceinte de l'autoclave à une température de stérilisation et pendant une durée de stérilisation, la température et la durée de stérilisation étant suffisantes pour stériliser la composition thermogélifiable ;
- diminuer la température de l'enceinte de l'autoclave ;
- sortir la composition thermogélifiable stérilisée de l'enceinte de l'autoclave.

Selon une variante la température de l'enceinte est maintenue à une température supérieure à 60°C, de préférence supérieure à 100°C, de préférence supérieure à 120°C.

Selon une variante, la température de stérilisation va de 120 à 140°C. Avantageusement, la durée de stérilisation est d'au moins 3 minutes, de préférence d'au moins 10 minutes, de préférence d'au moins 15 minutes et encore de préférence d'au moins 20 minutes.

La durée de stérilisation dépend en général du volume d'échantillon traité.

A titre indicatif, selon une variante, la durée de stérilisation est de 15 ou 20 minutes.

Plus spécifiquement, il est en général préconisé que la stérilisation soit effectuée pendant un maintien à une température de 121°C à 138°C pendant une durée de 3 à 30 minutes, conformément à la norme ISO17665.

Selon une variante, la température de stérilisation est maintenue à une température de 121 à 138°C pendant environ 20 minutes.

La surpression dans l'enceinte de l'autoclave est égale à la pression de la vapeur dans l'enceinte close. Elle est généralement supérieure ou égale à 2 bars pendant la durée de stérilisation. Encore plus précisément, il est préconisé que la stérilisation soit réalisée sous autoclave à une pression de 100kPa supérieure à celle atmosphérique pour une durée de 15 minutes ou équivalent (valeur F0).

Plus spécifiquement, la stérilisation à la vapeur a pour but d'éliminer tous les germes et/ou les contaminants.

Avantageusement, l'étape de refroidissement comprend le refroidissement jusqu'à la température ambiante (20-25°C). Avantageusement, cette étape comprend également la diminution de la pression jusqu'à la pression atmosphérique.

Selon une variante, le procédé de stérilisation comprend un ou plusieurs cycles de stérilisation à la vapeur.

On peut utiliser tout type d'autoclave convenant pour la stérilisation de produits destinés à être appliqués ou injectés dans le corps humain ou animal. De tels autoclaves sont connus de l'homme du métier.

Selon une variante, l'autoclave utilisé est un autoclave pour la stérilisation de produits biologiques et/ou thérapeutiques.

Avantageusement, la composition de l'invention est stérilisée dans un récipient, et plus particulièrement dans un dispositif permettant son injection dans le corps humain ou animal.

Avantageusement, pour remplir un dispositif permettant une injection, et plus spécifiquement une seringue, on peut appliquer une contre-pression à l'extrémité opposée de l'orifice de remplissage pour garantir le maintien du gel dans le dispositif, sans débordement.

Selon une variante, une seringue comprenant la composition de l'invention est placée dans l'enceinte d'un autoclave pour sa stérilisation.

On obtient ainsi une seringue stérilisée pré-remplie contenant la composition de l'invention stérilisée.

La présente invention concerne notamment des compositions thermogélifiables qui ne sont pas stérilisables par filtration, notamment car la stérilisation par filtration n'est pas adaptée à leur viscosité (sur filtre de $0{,}22\mu$m).

Selon une variante, les compositions thermogélifiables de l'invention présentent une viscosité supérieure à 200 mPa.s.

L'invention couvre encore une composition de l'invention lyophilisée. La lyophilisation peut intervenir avant et/ou après stérilisation.

L'invention couvre encore une composition de l'invention sous une forme sèche, notamment sous une forme lyophilisée.

On peut notamment (re)disperser cette composition lyophilisée avant usage.

La présente invention couvre également une méthode de traitement thérapeutique comprenant l'injection d'une composition selon l'invention.

La présente invention couvre également l'utilisation d'une composition selon l'invention pour la préparation d'une composition pharmaceutique, en particulier pour un traitement thérapeutique, par exemple comme défini plus spécifiquement par l'invention.

La présente invention couvre également une méthode de soin esthétique, en d'autres termes non-thérapeutique, comprenant l'utilisation ou l'injection d'une composition selon l'invention. Il s'agit par exemple du comblement de rides ou du comblement d'une ou plusieurs zones de tissu visible endommagées, par exemple suite à un accident ou une intervention chirurgicale, dans un but esthétique.

Un tissu est un ensemble de cellules semblables et de même origine, regroupées en ensemble fonctionnel, c'est-à-dire concourant à une même fonction. Parmi les tissus on peut citer : le tissu épithélial, le tissu conjonctif, le tissu musculaire, et le tissu nerveux.

On entend par « composition selon l'invention » ou des termes équivalents, une composition définie telle que dans la présente invention, y compris selon l'une quelconques des variantes, modes de réalisation particuliers ou spécifiques, indépendamment ou selon l'une quelconque de leurs combinaisons, y compris selon les caractéristiques préférées.

Sur les figures :

La figure 1 représente un spectre de résonance magnétique nucléaire du proton d'un chitosane succinamide de l'invention.

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

D'autre part, dans les exemples, tous les pourcentages sont donnés en masse, sauf indication contraire, et la température est exprimée en degré Celsius sauf indication contraire, et la pression est la pression atmosphérique, sauf indication contraire.

## Exemples

Les chitosanes précurseurs des chitosanes substitués selon l'invention présentent des masses moléculaires moyennes en viscosité (Mv, déterminées par viscosimétrie capillaire) et les degrés d'acétylation (DA, proportion d'unité N-acétyl-glucosamine, déterminée par titrage potentiométrique) compris dans les gammes du Tableau 1. La masse moléculaire d'un chitosane peut également être définie par la viscosité dynamique d'une solution de concentration 1% (m/m) en chitosane dans l'acide acétique de concentration 1% (v/v), mesurée par viscosimétrie à mobile tournant, par exemple un viscosimètre Brookfeld, comme décrit précédemment.

**Tableau 1.** Caractéristiques des chitosanes précurseurs des chitosanes substitués

| Gamme de masse moléculaire du chitosane | Gamme de Mv | Gamme de viscosité à 1% (mPa.s) | Gamme de DA (mol%) |
|---|---|---|---|
| ultra low | Environ 20 000 - 60 000 | 5 - 20 | 5 - 20% |
| low | Environ 60 000 - 120 000 | 20 - 50 | 15 - 25% |
| médium | Environ 120 000 - 150 000 | 50 - 80 | 20 - 30% |
| high | Environ 150 000 - 220 000 | 80 - 120 | 25 - 40% |

### Exemple 1 - Préparation d'un chitosane substitué (chitosane succinamide, CSS)

Pour obtenir le chitosane succinamide de référence CSS5 du Tableau 2, on dissout 10g de chitosane de masse moléculaire et de DA connus dans 266ml d'une solution aqueuse d'acide acétique à 1% (v/v). La solution est maintenue à une température de 30°C. 6,75g d'anhydride succinique (AS) sont ajoutés, ce qui correspond à un ratio massique d'anhydride succinique sur le chitosane (AS/chitosane) de 0/675, et à un ratio molaire d'anhydride succinique sur les groupes NH2 du chitosane (AS/NH2) de 1/7. Après 15 minutes, le pH du milieu réactionnel peut être éventuellement ajusté à 7,5 par ajout de soude 30%. La solution est ensuite précipitée dans 2,5 litres d'éthanol. Le précipité est récupéré, et de nouveau solubilisé dans l'eau. Le chitosane substitué subit ces étapes de précipitation dans l'éthanol puis solubilisation dans l'eau 3 fois. A la dernière précipitation, le précipité est récupéré, pressé et séché dans une étuve à 60°C à pression atmosphérique. Une fois le chitosane succinamide sec, il est broyé pour obtenir une poudre. Le degré de substitution du chitosane est déterminé par RMN du proton selon la méthode décrite précédemment. Un spectre de RMN est illustré par la Figure 1.

Les lots de chitosane succinamide utilisés dans les exemples ont été préparés de la même manière, avec les paramètres du Tableau 2.

**Tableau 2.** Paramètres et caractéristiques des lots de chitosane succinamide

| N° CSS | Gamme de Mv | Ratio massique AS/ chitosane | Ratio molaire AS/NH2 | DS (%mol) | Plage de pH pour insolubilité dans l'eau |
|---|---|---|---|---|---|
| CSS1 | médium | 0,119 | 0,3 | 12 | 5,4 - 6,7 |
| CSS2 | médium | 0,159 | 0,4 | 18 | 6,3 - 7,3 |
| CSS3 | médium | 0,199 | 0,5 | 25 | 6,1 - 7,0 |
| CSS4 | médium | 0,262 | 0,6 | 42 | |
| CSS5 | médium | 0,675 | 1,7 | 52 | < 5,5 |

(suite)

| N° CSS | Gamme de Mv | Ratio massique AS/ chitosane | Ratio molaire AS/NH2 | DS (%mol) | Plage de pH pour insolubilité dans l'eau |
|--------|-------------|------------------------------|----------------------|-----------|-------------------------------------------|
| CSS6 | médium | 0,178 | 0,45 | 21 | 5,8 - 6,9 |
| CSS7 | high | 0,19 | 0,5 | 17 | |
| CSS8 | high | 0,258 | 0,6 | 27 | 4,2 - 7,0 |
| CSS9 | high | 0,68 | 1,7 | 58 | < 5,2 |
| CSS10 | médium | 0,133 | 0,3 | / | 6,4 - 6,9 |
| CCS11 | médium | 0,133 | 0,3 | / | 5,9 - 7,1 |
| CSS12 | médium | 0,133 | 0,3 | / | 6,6 - 7,1 |
| CSS13 | médium | 0,133 | 0,3 | / | 6,5 - 7,2 |
| CSS14 | médium | 0,133 | 0,23 | / | 6,1 - 7,6 |
| CSS15 | médium | 0,133 | 0,23 | / | 4,8 - 6,3 |
| CSS16 | médium | 0,178 | 0,4 | 15 | 5,8 - 6,9 |
| CSS17 | médium | 0,222 | 0,5 | / | |
| CSS18 | médium | 0,172 | 0,4 | / | 5,5 - 7 |
| CSS19 | high | 0,2 | 0,5 | / | 5,2 - 6,3 |

**Tableau 2.** Paramètres et caractéristiques des lots de chitosane succinamide

Note : L'ajout de beta-glycérol phosphate de sodium (65%, Salfic-Alcan, en abrégé GP) permet de rendre le CSS soluble au-delà de la limite de solubilité dans l'eau. Le CSS présente un caractère zwiterionique. Cela ne présente de problème car on peut ajouter une faible concentration de GP pour retrouver le caractère soluble du CSS.

**Exemple 2 - Préparation d'un chitosane substitué (chitosane triméthylé, TMC)**

On prépare une suspension de 50g de chitosane dans un volume de 800ml d'eau. La suspension est chauffée à 95°C. Un volume de 26,27ml du réactif 3-chloro-2-hydroxypropyl trimethyl ammonium chloride (abréviation CHTAC) est ajouté goutte à goutte au milieu, soit un ratio massique et molaire CHTAC/chitosane de 0,31 et 0,5, respectivement. Après 4 heures de réaction, le milieu réactionnel est dilué avec 400ml d'eau, puis précipité dans 8,4 litres d'éthanol. Le précipité est récupéré et de nouveau solubilisé dans l'eau. Le chitosane substitué subit 3 fois cette étape de solubilisation/précipitation dans l'éthanol. A la dernière précipitation, le précipité est récupéré, pressé et séché dans une étuve à 60°C à pression atmosphérique. Une fois le chitosan triméthylé sec, il est broyé pour obtenir une poudre.

Les lots de chitosane triméthylé utilisés dans les exemples ont été préparés de la même manière, avec les paramètres du Tableau 3.

| N° TMC | Gamme de Mv du chitosane | Ratio massique CHTAC/ chitosane | Ratio molaire CHTAC/NH2 | DS (%mol) |
|--------|--------------------------|--------------------------------|--------------------------|-----------|
| TMC1 | médium | 0,37 | 0,5 | 16 |
| TMC2 | médium | 0,79 | 1,05 | 17 |
| TMC3 | médium | 0,37 | 0,5 | 22 |
| TMC4 | médium | 0,37 | 0,5 | 26 |
| TMC5 | médium | 0,37 | 0,5 | 28 |
| TMC6 | médium | 0,61 | 0,8 | 36 |
| TMC7 | médium | 1,21 | 1,5 | 75 |

**Tableau 3.** Paramètres et caractéristiques des lots de chitosane triméthylé

Note : Tous ces chitosanes triméthylés sont parfaitement solubles dans l'eau dans la gamme de DS de 16 à 75%.

**Exemple 3 - Exemple de préparation d'une composition thermogélifiable**

Un chitosane succinamide (CSS2) obtenu selon l'exemple 1 est solubilisé dans l'eau dans les proportions suivantes: 0,195g de chitosane succinamide CSS2 dans 14,809g d'eau contenant 0,5% (m/m) de mannitol. Une solution de beta-glycérol phosphate de sodium (65%, Salfic-Alcan, en abrégé GP) à 39,7% (m/m) est préparée. Sous agitation magnétique ou mécanique, 1,49ml de la solution de GP sont ajoutés à la solution de chitosane succinamide. 0,325ml de tampon acétate 0,5M (pH 3) est ajouté à la solution de chitosane succinamide/GP jusqu'à d'obtenir un pH donné, par exemple 7,5 dans le cas de la solution N°2 du Tableau 4. L'osmolalité de la solution finale est mesurée.

**Exemple 4 - Capacité à gélifier des solutions de chitosane succinamide à une concentration finale de 1,14% (m/m)**

Les solutions du Tableau 4 sont préparées selon la méthode décrite dans l'exemple 3.

**Tableau 4.** Solutions à base de CSS de Mv « médium » et « high » à une concentration de 1,14% (m/m), de GP à une concentration de 4,11% (m/m) et de mannitol à une concentration de 0,5% (m/m) ; le pH est ajusté à 7,5

| Gamme de Mv | N° solution | N° CSS | Ratio massique AS/ chitosane | Ratio molaire AS/NH2 | DS (%mol) | Facilité d'injection | Transition sol-gel à 37°C |
|---|---|---|---|---|---|---|---|
| médium | 1 | CSS1 | 0,12 | 0,3 | 12 | Oui | Non |
| | 2 | CSS2 | 0,16 | 0,4 | 18 | Oui | Oui |
| | 3 | CSS3 | 0,20 | 0,5 | | Oui | Oui |
| | 4 | CSS4 | 0,26 | 0,6 | 42 | Oui | Non |
| | 5 | CSS5 | 0,68 | 1,7 | 52 | Oui | Non |
| high | 6 | CSS6 | 0,18 | 0,4 | | Oui | Oui |
| | 7 | CSS7 | 0,19 | 0,5 | | Oui | Oui |
| | 8 | CSS8 | 0,26 | 0.6 | | Oui | Non |
| | 9 | CSS9 | 0,68 | 1,7 | | Oui | Non |

La capacité à gélifier de la solution thermogélifiable est dépendante en particulier du degré de substitution. Le degré de substitution est directement lié au rapport molaire entre les fonctions amine présentes sur le chitosane et la quantité d'anhydride succinique utilisée durant la réaction de substitution. Si le degré de substitution est trop élevé, la gélification n'a pas lieu (cas de la solution N°5). La gélification n'a pas lieu si le degré de substitution est trop faible (cas de la solution N°1).

Le Tableau 4 montre que pour le chitosane succinamide, le ratio massique AS/chitosane optimal conduisant au caractère thermogélifiable se trouve entre 0,12 et 0,26, ce qui se traduit par une gamme de DS de 10 à 45%.

Par ailleurs, les solutions thermogélifiables du Tableau 5 sont préparées au départ de lots différents de chitosane succinamide, pour démontrer la reproductibilité de la préparation des hydrogels avec un ratio massique de 0,13, qui permet de cibler un DS entre 14 et 18mol%. On voit que l'osmolalité de la solution finale se trouve entre 363 et 447mosm/kg, et que toutes les solutions sont faciles à injecter et thermogélifiables. La présence de mannitol (qui est optionnelle pour l'effet thermo-gélifiant) augmente l'osmolalité.

EP 3 156 059 B1

**Tableau 5.** Solutions à base de chitosane succinamide de Mv « médium » à une concentration de 1,14%, de GP à une concentration de 4,11% et mannitol à une concentration de 0,5%

| Gamme de Mv | N° solution | N° CSS | Osmolalité (mosm/kg) | Facilité d'injection | Gel à 37°C |
|---|---|---|---|---|---|
| medium | 10 | CSS10 | 363 | Oui | OK |
| | 11 | CSS11 | 382 | Oui | OK |
| | 12 | CSS12 | 447 | Oui | OK |
| | 13 | CSS13 | 405 | Oui | OK |
| | 14 | CSS14 | | Oui | OK |
| | 15 | CSS15 | | Oui | OK |

**Exemple 5 - Capacité du chitosane substitué TMC à gélifier à une concentration finale de 0.9% (m/m)**

Dans le cas du chitosane triméthylé de masse moléculaire « médium », le degré de substitution optimal pour la formulation de gel thermogélifiant, par exemple à une concentration faible de 0,9% (m/m), se trouve entre 10 et 36% (Tableau 6).

**Tableau 6.** Solutions à base de TMC à une concentration 0,9% (m/m) et de GP à une concentration de 3,0% (m/m) (pas de mannitol) ; le pH est ajusté à 7,5

| N° solution TMC | N° TMC | DA (mol%) | DS (mol%) | Gel à 37°C |
|---|---|---|---|---|
| 1 | TMC1 | 16 | 16 | Oui |
| 2 | TMC2 | 16 | 17 | Oui |
| 3 | TMC3 | 15 | 22 | Oui |
| 4 | TMC4 | 18 | 26 | Oui |
| 5 | TMC5 | 18 | 28 | Oui |
| 6 | TMC6 | 17 | 36 | Non |
| 7 | TMC7 | 19 | 75 | Non |

**Exemple 6 -** Capacité des solutions de chitosane succinamide à gélifier à faible concentration, à des valeurs d'osmolalité et pH physiologiques

On compare la solubilité, le caractère injectable et thermo-gélifiable des solutions à base de chitosane chitosane non substitué (CS) avec ceux des solutions à base de chitosane substitué (chitosane succinamide CSS), préparées selon la méthode de l'exemple 2 (Tableau 7).

**Tableau 7.** Solutions à base de chitosane (CS) ou de chitosane succinamide (CSS)

| CS ou CSS | Gamme de Mv | CS ou CSS (% m/m) | GP (%, m/m) | pH final (ajusté) | Facilité d'injection | Gel à 37°C |
|---|---|---|---|---|---|---|
| CS | high | 1,5% | 6% | 6,8-7,0 | Oui | Non |
| CS | high | 1,5% | 6% | 6,7-7,0 | Oui | Non |
| CS | high | 2% | 6% | 6,7-7,0 | Oui | Oui |
| CS | high | 2% | 4,1% | 6,7-7,0 | Oui | Non |
| CS | médium | 2% | 4,1% | 7,4 | Non (précipite) | Non |
| CS | low | 2% | 4,1% | 7,4 | Non (précipite) | Non |
| CS | médium | 1,5% | 4,1% | 7,4 | Non (précipite) | Non |

(suite)

| Tableau 7. Solutions à base de chitosane (CS) ou de chitosane succinamide (CSS) | | | | | | |
|---|---|---|---|---|---|---|
| CS ou CSS | Gamme de Mv | CS ou CSS (% m/m) | GP (%, m/m) | pH final (ajusté) | Facilité d'injection | Gel à 37°C |
| CS | low | 1,5% | 4,1% | 7,4 | Non (précipite) | Non |
| CSS | médium | 1,5% | 4,1% | 7,4 | Oui | Oui |

Avec le chitosane non substitué de l'art antérieur, on ne parvient pas à respecter le cahier des charges osmolalité, pH, solubilité dans l'eau, injectabilité et gélification à 37°C simultanément.

Avec le chitosane substitué par un groupe succinyle, on peut obtenir la solubilité, l'injectabilité et le caractère sol-gel même à plus faible concentration en GP (à concentration en polymère égale), grâce au fait que le polymère de départ est un chitosane substitué soluble dans l'eau, la concentration en GP étant modulable pour obtenir le caractère gélifiable, l'osmolalité et le pH désirés.

**Exemple 7 -** Capacité des solutions de chitosane succinamide de concentration finale 0,88% à gélifier sur une plage de pH large

Dans cet exemple, le pH des solutions a été ajusté par ajout d'acide acétique concentré (96% m/v) afin de minimiser la dilution de l'hydrogel. Toutes les solutions sont solubles et facilement injectables.

| Tableau 8. Solution à base de CSS à une concentration de 0,88% (m/m) et de GP à une concentration de 4,16% | | | |
|---|---|---|---|
| Gamme de Mv | N° CSS | pH | Gel à 37°C |
| médium | CSS10 | 6,8 | Non |
| | | 7,0 | Non |
| | | 7,2 | Oui |
| | | 7,5 | Oui |
| | | 7,6 | Oui |
| | | 8,0 | Oui |

Pour une masse moléculaire et un degré de substitution donnés, il est possible d'obtenir le caractère gélifiant sur une plage de pH large, par exemple de 7,2 à 8,0, par exemple au départ d'un CSS de masse moléculaire « médium » et d'un ratio massique de 0,133.

**Exemple 8 -** Capacité à diminuer la concentration en chitosane substitué (CSS) tout en conservant la capacité à gélifier de la solution

| Tableau 9. Solutions à base de CSS de faible concentration | | | | | | |
|---|---|---|---|---|---|---|
| Gamme Mv | N° CSS | CSS (% m/m) | pH | Gamme de concentration en GP (% m/m) | Gamme d'osmolalité | Gel à 37°C |
| high | CSS7 | 0,75% | 7,0 | 3,5 - 4,1 | 310 - 376 | Non |
| | | | 7,5 | 3,4 - 4,2 | 300 - 375 | Oui |
| | | 0,88% | 7,0 | 3,4 - 4,4 | 300 - 373 | Non |
| | | | 7,5 | 3,4 - 4,4 | 300 - 380 | Oui |
| | | 1,34% | 7,0 | 3 - 3,7 | 300 - 380 | Oui |

(suite)

| Gamme Mv | N° CSS | CSS (% m/m) | pH | Gamme de concentration en GP (% m/m) | Gamme d'osmolalité | Gel à 37°C |
|---|---|---|---|---|---|---|
| médium | CSS17 | 0,88% | 7,0 | 3,4 - 3,8 | 300 - 375 | Non |
| | | | 7,5 | 3,4 - 4,5 | 300 - 400 | Non |
| | | 1,35% | 7,0 | 2,4 - 2,7 | 300 - 360 | Non |
| | | | 7,5 | 2,4 - 2,7 | 300 - 340 | Oui |

**Tableau 9.** Solutions à base de CSS de faible concentration

Il est parfois avantageux de former les solutions de faibles concentrations finale en chitosane substitué dans certaines applications, par exemple en dessous de 1%, par exemple 0,85%, tout en conservant la capacité à produire un gel à température physiologique, par exemple 7,5.

**Exemple 9** - **Injectabilité des produits commerciaux et des solutions de chitosane substitué et GP à température ambiante**

Cet exemple vise à vérifier que le produit peut être facilement injecté à travers une seringue équipée d'une aiguille de faible diamètre, telle qu'utilisée pour les injections par exemple en intra-dermique ou en intra-articulaire. En particulier, il est important de pouvoir injecter facilement dans des petites articulations comme celles de la main.

On évalue la force nécessaire à injecter les solutions à température ambiante au cours de l'injection de la solution via une aiguille de 22 Gauge, selon le protocole décrit dans le texte. On mesure la force nécessaire à l'injection après un déplacement du piston de la seringue d'environ 15mm, ce qui permet aisément de comparer les différents produits entre eux. Trois solutions d'acide hyaluronique destinées à l'injection intra-articulaire disponibles commercialement ont été testées, et comparées avec des solutions de chitosane succinamide de masse moléculaire « médium » et de concentration variable.

**Tableau 10.** Force nécessaire pour l'injection après un déplacement du piston d'environ 15mm pour une aiguille 22 Gauge

| Produit | Force (N) |
|---|---|
| Eau pure | 0,5 |
| Solution commerciale « A » (acide hyaluronique) | 2 N |
| Solution commerciale « C » (acide hyaluronique) | 6 - 8 N |
| Solution à base de CSS18 à une concentration de 1,0 % à pH 7,5 | 3 N |
| Solution à base de CSS19 à une concentration de 1,5% à pH 7,5 | 3 N |

On voit que les solutions de chitosane succinamide sont aussi facilement injectable à travers une aiguille de 22 Gauge qu'une solution commerciale d'acide hyaluronique (A), et plus facilement injectables qu'une solution commerciale d'acide hyaluronique (C).

**Exemple 10 - Propriétés rhéologiques des produits commerciaux à base d'acide hyaluronique et des solutions thermogélifiables à base de chitosane succinamide**

On mesure les modules G' et G" de produits commerciaux à base d'acide hyaluronique à une température allant de de 4°C à 37°C, en fonction du temps, à l'aide d'un rhéomètre ARES, selon la méthode décrite précédemment. Puis on détermine la valeur des modules G' et G" à 60 minutes (Tableau 11).

| Tableau 11. Modules G' et G" à 60 minutes | | | | |
|---|---|---|---|---|
| Produit | Sol ou gel à 4°C | G' à 37°C (Pa) | G" à 37°C (Pa) | Sol ou gel à 37°C (G' versus G") |
| Solution commerciale (acide hyaluronique) « A » | Sol | 0.1 | 1 | Sol (G' < G") |
| Solution commerciale (acide hyaluronique) « B » | Gel | 40 | 38 | Gel (G' > G") |
| Solution commerciale (acide hyaluronique) « D » | Gel | 640 | 120 | Gel (G' > G") |
| Solution à base de CSS16 à une concentration de 1,2% à pH 7,5 (solution de l'exemple 12) | Sol | 16 | 1 | Gel (G' > G") |

Pour les compositions de l'invention, par exemple pour la composition à base de chitosane succinamide et de GP à pH=7,5, le module G' croise le module G" au bout de quelques secondes, et G' est supérieur à G" au- delà du temps auquel le croisement s'est produit, caractérisant une transition sol-gel rapide lorsque le produit passe de 4°C à 37°C.

Les compositions commerciales à base d'acide hyaluronique testées ne sont pas thermogélifiables.

**Exemple 11** - **Effet de l'injection intra-articulaire d'une solution de chitosane succinamide chez le lapin après transection du ligament antérieur**

La solution de cet exemple est une solution stérilisée comprenant 1,2% (m/m) de chitosane succinamide de degré de substitution 15% et de masse moléculaire « médium » (N° CSS16 du Tableau 2), 3,5% (m/m) de GP, 0,4% (m/m) de D-mannitol et 11mmol/l de sodium acétate trihydrate. La solution est stérilisée à la vapeur par autoclave, la concentration est ajustée par dilution d'un facteur de 1,33, puis la solution est conditionnée dans une seringue. Après stérilisation et dilution, le pH est égal à 7,5, l'osmolalité est égale à 380mosm/kg, et la viscosité dynamique apparente est égale à 126mPa.s à 9°C.

Une transection du ligament antérieur, appelée « anterior cruciate ligament transection (ACLT)», chez le lapin entraîne de manière consistante et bien documentée des modifications symptômatiques de l'ostéoarthrose (OA), à savoir des fissures du cartilage, des lésions et une inflammation de la membrane synoviale importantes sans perte de cartilage sur toute la hauteur de l'articulation. Ce modèle est utilisé en particulier pour étudier les effets de nouvelles thérapies comme les viscosuppléments injectés intra-articulairement sur la structure du cartilage, les lésions de départ pouvant être d'origine et de nature très variables (in Laverty et al. Osteoarthr Cartil, 2010 ; Edouard et al. Phys Ther Sport, 2013 ; Mainil-Varlet et al. Cartilage, 2013; Oprenyeszk et al. Osteoarthr Cartil, 2013).

Dans ce modèle, une érosion du cartilage apparaît dès 4 semaines après l'intervention chrirugicale qui consiste à effectuer une transection du ligament antérieur du genou droit. Il est recommandé de mener l'étude jusqu'à 8 semaines après l'intervention, ce qui permet d'obtenir une érosion du cartilage dans au moins 40% des condyles fémoraux. L'injection intra-articulaire du produit à tester se fait avant l'apparition des premiers signes d'ostéroarthrose, c'est à dire 1 semaine après la chirurgie sur le genou ayant subi la transection. Le genou droit des 10 animaux du groupe test a reçu une injection de 600µl de la solution thermogélifiable à tester. Le genou droit des 9 animaux du groupe « contrôle » a reçu une injection de 600µl de solution saline à 0.9%. Les produits sont injectés dans l'articulation du genou des lapins via une seringue munie d'une aiguille de de 22 Gauge.

On caractérise la capacité du produit à ralentir ou stopper la progression de l'arthrose induite par la chirurgie par l'examen de plusieurs indicateurs macroscopiques, histologiques and radiologiques, caractérisés selon les scores recommandés par l'OARSI (in Laverty et al. Osteoarthr Cartil 2010). Les résultats de la radiologie des articulations sont évalués selon l'échelle de Kellgren & Lawrence, sur base de la présence d'ostéophytes et de l'espace inter-articulaire.

L'étude se fait en double aveugle, c'est-à-dire que ni le chirurgien qui procède à l'opération et à l'injection des produits à tester, ni les personnes qui réalisent les observations macroscopiques au cours de la vie des animaux et au moment de leur euthanasie, ni les personnes qui analysent les résultats macroscopiques ou histologiques et établissent les calculs statistiques ne connaissent la correspondance entre les numéros des animaux et les groupes par produit testé. On ne communique cette correspondance qu'une fois les calculs terminés et rédigés par une personne indépendante. Les résultats sont rapportés dans les tableaux 11 à 13.

Par rapport à l'injection d'une solution saline, l'injection de la solution thermogélifiable HG permet de diminuer de manière statistiquement significative tous les indicateurs de ostéoarthrose à 8 semaines, comme démontré par une diminution de l'espace inter-articulaire et de la présence d'ostéophytes visibles à l'examen radiologique (Tableau 12, selon l'échelle de Kellgren & Lawrence), la diminution de la sévérité et la taille des lésions du cartilage qui apparaîssent sur le côté latéral des articulations (Tableau 13), et la diminution des infiltrats inflammatoires de la membrane synoviale (Tableau 14). Aucun effet secondaire indésirable de la solution thermogélifiable n'est détecté par rapport au groupe contrôle, traduisant la bonne tolérance du produit dans les conditions de l'étude.

**Tableau 12.** Score radiologique selon l'échelle de Kellgren & Lawrence (de 0 à 4) évaluant l'espace inter-articulaire et la présence d'ostéophytes (valeur-p calculée par la méthode non-paramétrique du test U de Mann-Whitney)

| Score radiologique selon K&L (0-4) | Solution saline | Solution thermogélifiable |
|---|---|---|
| Nombre d'animaux | 9 | 10 |
| N observation | 9 | 10 |
| Score moyen | 2,0 | 0,0 |
| Score médian | 1,8 | 0,4 |
| Score min | 0,0 | 0,0 |
| Score max | 3,0 | 3,0 |
| Ecart-moyen | 0,7 | 0,6 |
| Différence entre les 2 groupes et valeur-p (probabilité) | - | Statistiquement significative Valeur-p = 0,0079 |

**Tableau 13.** Score macroscopique global évaluant la taille et la sévérité des lésions des compartiments latéraux du cartilage des plateaux tibiaux et condyles fémoraux (valeur-p calculée par la méthode non-paramétrique du test U de Mann-Whitney)

| Score macroscopique global (taille et sévérité des lésions) | Solution saline | Solution thermogélifiable |
|---|---|---|
| Nombre d'animaux | 9 | 10 |
| N observation | 18 | 20 |
| Score moyen | 32,0 | 17,5 |
| Score médian | 32,8 | 21,4 |
| Score min | 3,0 | 3,0 |
| Score max | 68,0 | 44,0 |
| Ecart-moyen | 13,4 | 10,2 |
| Différence entre les 2 groupes et valeur-p (probabilité) | - | Statistiquement significative Valeur-p < 0,0041 |

**Tableau 14-** Score de l'infiltrat inflammatoire de la membrane synoviale évalué par histologie (valeur-p calculée par la méthode non-paramétrique du test U de Mann-Whitney)

| Score de l'infiltrat inflammatoire (0-5) | Solution saline | Solution thermogélifiable |
|---|---|---|
| Nombre d'animaux | 9 | 10 |
| N observation | 27 | 28 |
| Score moyen | 2,0 | 1,4 |
| Score médian | 2,6 | 1,8 |
| Score min | 1 | 0,5 |
| Score max | 5 | 5,0 |
| Ecart-moyen | 1,0 | 1,0 |

(suite)

| Tableau 14- Score de l'infiltrat inflammatoire de la membrane synoviale évalué par histologie (valeur-p calculée par la méthode non-paramétrique du test U de Mann-Whitney) | | |
|---|---|---|
| Différence entre les 2 groupes et valeur-p (probabilité) | | Statistiquement significative Valeur p = 0,0011 |

**Exemples 12 - 24**

Dans les exemples 12 à 24, les méthodes et les protocoles suivants ont été utilisés :

Propriétés rhéologiques

Les propriétés rhéologiques sont mesurées à l'aide d'un rhéomètre « Discovery Hybrid DHR-2 » (TA Instrument ; voir par exemple : http://www.tainstruments.com), équipé avec une plaque Peltier (Peltier Plate) et un piège à solvants. La géométrie utilisée est la plaque Peltier en acier de diamètre 25mm, et la distance entre le plateau et le Peltier est fixée à 0,7mm. Les mesures sont réalisées à 37$\underline{o}$C, sauf pour l'expérience de balayage en température décrite à l'exemple 12. On réalise les mesures selon les protocoles suivants :

- Identification de la région linéaire viscoélastique par balayage en déformation

Un test de balayage en déformation est d'abord réalisé pour identifier la région linéaire viscoélastique (LVER) de chaque produit, à 37°C.

- Balayage en fréquence en mode oscillatoire faible

Un balayage en fréquence entre 0.01rad/s et 100 rad/s est réalisé dans la région « LVER » de chaque produit, à une déformation de 0,9%, avec le mode oscillatoire de faible amplitude (« small amplitude oscillatory shear », SAOS) à 37°C. Les caractéristiques suivantes sont mesurées en fonction de la fréquence de balayage : module d'élasticité (G'), module de perte (G"), fréquence de croisement des modules G' et G" (« fréquence de crossover ») et viscosité complexe ($\eta$*). En particulier, on détermine la valeur du module d'élasticité G' à la fréquence de 0,6Hz, qui correspond à la fréquence appliquée au liquide synovial du genou lors de la marche.

- Balayage en écoulement

On détermine la viscosité au repos via le mode de balayage en écoulement. Le produit est équilibré pendant 1 minute à 37°C. Un taux de cisaillement de 0,001 s-1 à 10 s-1 est appliqué pour mesurer la viscosité en fonction du taux de cisaillement. A partir de la courbe obtenue sur cette gamme de taux de cisaillement, on détermine la viscosité au repos en extrapolant la valeur de la viscosité à cisaillement nul. Dans le cas où le coefficient de corrélation est inférieur à 0,98, la viscosité au plus faible taux de cisaillement (0,001 s-1) est rapportée.

- Degré de substitution (mesure RMN)

Le degré de substitution est déterminé dans les exemples qui suivent par spectrométrie de résonance magnétique (RMN) du proton en solution en milieu aqueux, à l'aide d'un spectromètre de résonance magnétique, par exemple un spectromètre Bruker de fréquence 400 MHz. Les échantillons sont préparés de la manière suivante : 5 à 6mg de chitosane substitué sont dissout dans 1ml d'eau deutérée. On ajoute 2$\mu$l d'acide chlorhydrique deutéré ou de borate de sodium à la solution de chitosane substitué afin d'atteindre une zone de pH appropriée pour l'analyse. La zone de pH appropriée dépend de la nature du substituant et peut être de 8,5 par exemple avec le borate de sodium et entre 3 et 4 avec HCI 12M. Le spectre est enregistré à une température de 70°C, avec un nombre de scans allant de 64 à 256 et un temps de relaxation allant de 1 à 8 secondes. Le spectre obtenu est traité par déconvolution afin de déterminer la valeur de l'intégrale des aires des signaux d'intérêt pour pouvoir calculer le degré de substitution du chitosane substitué.

Les chitosanes substitués qui ont été utilisés dans les exemples 12 à 24 ont été préparés et caractérisés selon les méthodes décrites précédemment (tableau 15). La masse moléculaire du chitosane est définie par la viscosité d'une solution de concentration en chitosane de 1% (m/m) dans l'acide acétique de concentration 1% (v/v).

Tableau 15 - Caractéristiques des chitosanes substitués et des chitosanes précurseurs de ces chitosanes substitués

| Chitosanes substitués | | Chitosanes précurseurs | | |
|---|---|---|---|---|
| Référence | DS (mol%) | Référence | Range Mw | Viscosité à 1% (mPa.s) |
| Chitosane succinamide | | | | |
| CSS20 | 29** | CS1 | Médium | 40 |
| CSS21 | 18* | CS2 | | 60 |
| CSS22 | 19** | CS3 | | 63 |
| CSS23 | 19** | | | |
| CSS24 | 20** | CS4 | | 72 |
| CSS25 | 20* | CS5 | | 78 |
| CSS26 | 20* | | | |
| CSS27 | 25* | | | |
| CSS28 | 30* | | | |
| CSS29 | 20** | | | |
| CSS30 | 28** | CS4 | | 72 |
| Triméthyl-chitosane | | | | |
| TMC8 | 26** | CS2 | Médium | 60 |
| *Degré de substitution estimé sur base de la valeur ciblée lors de la réaction de substitution; **degré de substitution mesuré par RMN du proton selon la méthode décrite précédemment. | | | | |

Dans les exemples qui suivent, les formulations de chitosane substitué sont préparées selon le protocole suivant :

- Solubiliser la poudre de chitosane substitué à la concentration voulue contenant la concentration désirée de sorbitol ou de mannitol ;
- Ajouter une solution-mère de tampon phosphate (100mM) afin d'obtenir une osmolalité comprise entre 280 et 400mosm/kg ;
- Ajouter de l'acide acétique glacial, jusqu'à obtenir un pH entre 7,2 et 8,5.

Tous les exemples suivants concernent des formulations stérilisées par autoclave, sauf lorsqu'il est mentionné « avant autoclave », selon le protocole suivant :

- Mettre la solution dans un contenant approprié et stériliser par autoclave à 121°C durant 20 minutes.

Le tableau 16 contient la liste des formulations à base de chitosane substitué décrites dans les exemples 12 à 22.

Tableau 16 - Formulations à base de chitosane substitué

| Formulation (référence) | Chitosane substitué | | Acide hyaluronique | | GP | Sucre | |
|---|---|---|---|---|---|---|---|
| | Réf | Concentration (%, m/m) | Réf | Concentration (%, m/m) | | Mannitol ou sorbitol | Concentration (%, m/m) |
| Chitosane succinamide | | | | | | | |
| 17-008 | CSS29 | 2% | / | / | / | Sorbitol | 0,5% |
| 16-27A | | 1,75% | / | / | / | Sorbitol | 0,5% |
| 16-27B | | 1,5% | / | / | / | Sorbitol | 0,5% |

(suite)

| Formulation (référence) | Chitosane substitué | | Acide hyaluronique | | GP | Sucre | |
| --- | --- | --- | --- | --- | --- | --- | --- |
| | Réf | Concentration (%, m/m) | Réf | Concentration (%, m/m) | | Mannitol ou sorbitol | Concentration (%, m/m) |
| 8-147D | CSS2 | 2% | / | / | / | Mannitol | 0,5% |
| 17-032-1 | CSS26 | 2% | / | / | / | Sorbitol | 0,5% |
| 17-032-2 | | 1,25% | / | / | / | Sorbitol | 0,5% |
| 17-033C-1 | CSS27 | 2% | / | / | / | Sorbitol | 0,5% |
| 17-033C-2 | | 1,25% | / | / | / | Sorbitol | 0,5% |
| 17-033B-1 | CSS28 | 2% | / | / | / | Sorbitol | 0,5% |
| 17-033B-2 | | 1,25% | / | / | / | Sorbitol | 0,5% |
| 8-145A | CSS25 | 2% | / | / | / | Mannitol | 0,5% |
| 8-147B | | 2% | / | / | / | Mannitol | 0,5% |
| 8-147C | | 2% | / | / | / | Mannitol | 0,5% |
| 16-008A | CSS23 | 2% | | | / | Sorbitol | 0,5% |
| 16-008B | | 2% | / | / | Oui | Sorbitol | 0,5% |
| 14-084 | CSS29 | 1% | H34 | 1% | / | / | / |
| 14-49A1 | CSS25 | 0,33% | H34 | 1,67% | / | / | / |
| 14-49A3 | | 0,5% | | 1,5% | / | / | / |
| 14-49A2 | | 0,67% | | 1,33% | / | / | / |
| 14-49A4 | | 1,0% | | 1,0% | / | / | / |
| 14-49A5 | | 0,5% | H22 | 1,5% | / | / | / |
| 14-49A6 | | 1,0% | | 1,0% | / | / | / |
| 013-009 | CSS21 | 1% | / | / | Oui | / | / |
| 13-006E4 | | 1,3% | / | / | Oui | / | / |
| 13-010 | | 1,5% | / | / | Oui | / | / |

**Exemple 12** - **Caractère thermo-gélifiant de formulations à base de chitosane succinamide**

Un balayage en température est réalisé afin de suivre l'évolution des modules d'élasticité (G') et de perte (G")
de différentes formulations à base de chitosane succinamide en fonction de la température (tableau 17), à l'aide du
rhéomètre « Discovery Hybrid DHR-2 » (TA Instrument) équipé avec un Peltier et une géométrie de type plateau parallèle
en acier de 25mm, avec une distance de 0,7mm. La montée en température est précédée d'un pré-cisaillement au fin
de standardiser les différences entre échantillons pendant la mise en place de l'échantillon sur le rhéomètre. La déformation, la vitesse de la montée en température et la fréquence sont ajustés en fonction de chaque échantillon testé
(tableau 17).

Tableau 17 - Module d'élasticité (G') et de module de perte (G") de formulations à base de chitosane succinamide à différentes températures

| Référence (formulation) | Pré-cisaillement (fréquence, durée) | Déformation (%) | Fréquence (rad/s) | Montée en température (°C/min) | G' ; G" (Pa) | | | | |
|---|---|---|---|---|---|---|---|---|---|
| | | | | | 5°C | 10°C | 20°C | 25°C | 37°C |
| | | | | | | | | | |
| 17-008 | 10 rad/s 30s | 250% | 2,5 | 5 | G '= 29 G"=23 | G '= 34 G"=32 | G '= 43 G"=23 | G '= 47 G"=25 | G '= 54 G"=36 |
| 16-27A | 25 rad/s 90s | 160% | 3 | 5 | G '= 9 G"=7 | G '= 10 G"=7 | G '= 14 G"=6 | G '=16 G"=6 | G '=23 G"=7 |
| 16-27B | 25 rad/s 90s | 160% | 3 | 5 | G '=6 G"=7 | G '= 8 G"=7 | G '= 11 G"=6 | G '=13 G"=5 | 5G '= 21 G"=5 |
| 14-084 | 20 rad/s 120s | 25% | 0,1 | 1,5 | G '= 46 G"=51 | G '= 51 G"=50 | G '= 52 G"=48 | G '= 52 G"=47 | G '= 62 G"=42 |

On comprend de cet exemple que pour toutes les formulations à base de chitosane succinamide testées, le module d'élasticité G' devient supérieur au module d'élasticité G" à partir d'une certaine température, ce qui montre le caractère thermo-gélifiant des formulations. La température à laquelle G' croise G" varie en fonction de la composition de la formulation et des caractéristiques moléculaires du chitosane succinamide.

**Exemple 13** - **Formulations de faible concentration en chitosane substitué, en vue de l'injection au travers d'aiguilles très fines (29G et 30G)**

Les modules d'élasticité (G') et de perte (G") de formulations à base de chitosanes substitués (succinamide et triméthylé) sont mesurés à l'aide d'un rhéomètre ARES avec une géométrie Peltier « cone-plate » à une distance de $50\mu m$ et un angle de 0,04 radian, à une fréquence de 10Hz à 37°C, avec une déformation de 3%, selon la méthode décrite précédemment. (Tableau 18a) La viscosité dynamique est mesurée à l'aide d'un viscosimètre à mobile tournant (Brookfield), comme décrit précédemment (tableau 18a). La force nécessaire pour l'injection des formulations au travers d'aiguilles 29 et 30 Gauge est mesurée comme décrit précédemment (tableau 18b).

Tableau 18a - Propriétés rhéologiques et viscosité

| Formulation (Référence) | Type | Gel (G'>G") | G' à 900s (Pa) | Viscosité dynamique (mPa.s) |
|---|---|---|---|---|
| 13-009 | CSS 1% | Gel après 40s | 100 | 439 |
| 13-006E4 | CSS 1,3% | Gel | 153 | 744 |
| 13-010 | CSS 1,5% | Gel | 95 | 1621 |
| 13-008 | TMC 1,1% | Gel | 70 | 241 |
| 13-006F4 | TMC 1,3% | Gel | 137 | 418 |

Tableau 18b - Injectabilité (force nécessaire pour l'injection) en Newton

| Réf | Taille de l'aiguille | |
|---|---|---|
| | 29G | 30G |
| | Thin-walled | Normal-walled |
| 13-009 | 3,6N | 7,5N |
| 13-006E4 | 6N | 11N |
| 13-010 | 5,75N | 12,5N |
| 13-008 | 2,7N | 5,5N |
| 13-006F4 | 4,1N | 8,9N |

Il ressort que l'on obtient des formulations facilement injectables au travers d'aiguilles très fines, adaptées par exemple aux injections sous-cutanées, intra-dermales, ou encore aux micro-injections pratiquées au niveau de l'épiderme notamment pour la réjuvénation de la peau, ou encore aux injections intra-camérulaires pour l'ophtalmologie, tout en conservant les propriétés de gel avec une bonne élasticité et viscosité.

**Exemple 14** - **Propriétés rhéologique de viscosuppléments commerciaux**

On mesure les propriétés rhéologiques de produits commerciaux à base d'acide hyaluronique de type non réticulé (VS1 et VS2) ou réticulé par des liaisons covalents (VS3), destinés à la viscosupplémentation, à l'aide à l'aide du rhéomètre « Discovery Hybrid DHR-2 » (TA Instrument) à 37°C en mode oscillatoire, selon les méthode décrite précédemment. On détermine la fréquence de croisement (« crossover ») entre les modules G' et G", la valeur de G' à la fréquence de 0,6Hz qui correspond à la fréquence de marche. On détermine la viscosité au repos en mode de balayage en écoulement, selon la méthode décrite précédemment.

Tableau 19 - Propriétés rhéologique de viscosuppléments commerciaux

| Référence | Type de réticulation | Concentration HA | Fréquence de crossover (Hz) | G' à 0.6Hz (Pa) | Viscosité au repos (Pa.s) |
|---|---|---|---|---|---|
| VS1 | Pas de réticulation | 2% | 5,2 Hz | 114 | 212 |
| VS2 | Pas de réticulation | 1% | Pas de croisement | 0,05 | 7 |
| VS3 | Réticulation covalente | 0,8% | Pas de croisement | 114 | 835 |

On comprend de cet exemple que le comportement rhéologique des viscosuppléments à base d'acide hyaluronique dépend de leur composition et des caractéristiques moléculaires de l'acide hyaluronique, notamment de la structure réticulée par des liaisons covalentes ou non.

**Exemple 15** - **Formulations de chitosane succinamide à différentes concentrations en glycérophosphate**

On mesure les propriétés rhéologiques de formulations à base de chitosane succinamide de masse moléculaire « médium » (référence CSS25) à la concentration de 2% (m/m) et de glycérophosphate (GP) de concentration variable, à l'aide à l'aide du rhéomètre « Discovery Hybrid DHR-2 » (TA Instrument) à 37°C selon les méthodes décrites précédemment (tableau 20).

Tableau 20 - Propriétés rhéologiques de formulations à base de chitosane succinamide à 2% (m/m) en fonction de la concentration en GP

| Formulation (référence) | GP % (m/m) | G'>G" à 0,6Hz | Fréquence de crossover | G' à 0.6Hz (Pa) | Viscosité au repos (mPa.s) |
|---|---|---|---|---|---|
| 8-145A | 4% | | | 526 | 8160 |
| 8-147B | 2% | Oui | Pas de croisement | 298 | 29000 |
| 8-147C | 1% | | | 164 | 14460 |
| 8-147D | 0% | | | 184 | 12034 |

Il ressort de cet exemple que :

- Une solution de chitosane succinamide de degré de substitution de 20% et une masse moléculaire de type « médium » peut former un gel sans ajout de GP ;
- On peut augmenter la cohésion du gel en ajoutant du GP en concentration croissante ;
- La capacité de lubrification (viscosité au repos) atteint un maximum lorsqu'on augmente la proportion en GP.

**Exemple 16** - **Formulations à base de chitosane succinamide de degré de substitution et de concentration variables**

Des solutions de chitosane succinamide de concentration variable sont préparées comme décrit précédemment, au départ de chitosane succinamode de degré de substitution (DS) différents (référence CSS26, CSS27, CSS28) et de même masse moléculaire (« médium »), sans GP. On mesure leurs propriétés rhéologiques des formulations à l'aide à l'aide du rhéomètre « Discovery Hybrid DHR-2 » (TA Instrument) à 37°C selon les méthodes décrites précédemment (tableau 21).

Tableau 21 - Propriétés rhéologique de formulations à base de chitosane succinamide en fonction du DS

| Formulation (référence) | Concentration (%, m/m) | CSS (référence) | DS* (mol%) | G'>G'' à 0,6Hz | G' à 0.6Hz (Pa) | Viscosité au repos (mPa.s) | Fréquence de Crossover |
|---|---|---|---|---|---|---|---|
| 17-032-1 | 2% | CSS26 | 20% | Oui | 299 | 10500 | Pas de croisement |
| 17-032-2 | 1,25% | | | | 58 | 3400 | |
| 17-033C-1 | 2% | CSS27 | 25% | | 52 | 2500 | |
| 17-033C-2 | 1,25% | | | | 9 | 200 | |
| 17-033B-1 | 2% | CSS28 | 30% | | 30 | 1000 | |
| 17-033B-2 | 1,25% | | | | 10 | 130 | |
| *DS estimé comme décrit précédemment | | | | | | | |

Il ressort que :

- On obtient des gels pour toutes les formulations préparées au départ de CSS de DS de 20, 25 et 30%;
- On module la cohésion et la viscosité au repos en variant le DS et la concentration en CSS, pour une même masse moléculaire (médium).

**Exemple 17** - **Formulations à base de chitosane succinamide et d'acide hyaluronique**

Une solution de chitosane succinamide (référence CSS25) à 2% (m/m) est préparée comme décrit précédemment. Une solution d'acide hyaluronique (HA) à 2% (m/m) est préparée en solubilisant HA et le mannitol dans un tampon phosphate, pour obtenir une osmolarité entre 280 et 400 mosm/kg et un pH entre 7,2 et 8,5. On utilise un HA de haute masse moléculaire (référence H34, viscosité intrinsèque de 3m$^3$/kg, telle que fournie par le fabricant) ou de faible masse moléculaire (référence H22, viscosité intrinsèque de 1,55m3/kg). Les solutions sont ensuite mélangées dans différentes proportions en masse. Elles sont stérilisées par autoclave comme décrit précédemment. On mesure leurs propriétés rhéologiques des formulations à l'aide à l'aide du rhéomètre « Discovery Hybrid DHR-2 » (TA Instrument) à 37°C selon les méthodes décrites précédemment (tableau 22).

Tableau 22 - Propriétés rhéologique de formulations à base de chitosane succinamide et d'acide hyaluronique

| Formulation (référence) | HA (référence) | Conc. en CSS (%, m/m) | Conc. en HA (%, m/m) | Fréquence de crossover | G' à 0,6Hz (Pa) | Dynamic viscosity at 0,6Hz (mPa.s) |
|---|---|---|---|---|---|---|
| 8-147D | / | 2,0% | / | Pas de croisement | 650 | 164 |
| 49-A1 | H34 | 0,33% | 1,67% | 1,2 Hz | 48 | 19 |
| 49-A3 | | 0,5% | 1,5% | 1,2 Hz | 36 | 14 |
| 49-A2 | | 0,67% | 1,33% | 1,1 Hz | 35 | 14 |
| 49-A4 | | 1,0% | 1,0% | Pas de croisement | 47 | 15 |
| 49-A5 | H22 | 0,5% | 1,5% | 13 | 7 | 5 |
| 49-A6 | | 1,0% | 1,0% | 0,1 | 13 | 5 |

On comprend des résultats que les propriétés rhéologiques des gels formés par le mélange CSS et HA sont modulables avec la concentration, la proportion entre CSS et HA et la viscosité intrinsèque du HA. Le HA de haute viscosité permet d'obtenir le gel qui a les G' et viscosité dynamique les plus élevées à la fréquence de marche (0,6Hz). De plus, dans certaines conditions, on n'a pas de croisement des modules G' et G'', montrant qu'un gel est formé quelle que soit la fréquence de cisaillement.

**Exemple 18** - **Synergie avec le liquide synovial du genou de patients arthrosiques**

Avec leur consentement, on prélève le liquide synovial (SF) de quatre patients adultes volontaires atteints d'ostéo-arthrose du genou avant l'implantation d'une prothèse (SF1 à SF4). On le mélange avec différentes solutions à base de chitosane succinamide ou avec différents viscosuppléments commerciaux dans un ratio massique de 1/1, puis on mesure les propriétés rhéologiques des mélanges à l'aide à l'aide du rhéomètre « Discovery Hybrid DHR-2 » (TA Instrument) à 37°C selon les méthodes décrites précédemment (tableau 23).

Tableau 23 - Propriétés rhéologiques du liquide synovial du genou de quatre patients atteints d'ostéo-arthrose, et de formulations de chitosane succinamide ou de viscosuppléments commerciaux, avant et après mélange avec le liquide synolvial à un ratio volumique de 50/50 (v/v).

| Formulation seule | | | Formulation mélangée au liquide synovial | | | | |
|---|---|---|---|---|---|---|---|
| Formulation (référence) | G' à la fréquence 0,6Hz (Pa) | Viscosité au repos (Pa.s) | SF (référence) | G'>G'' à la fréquence 0,6Hz | G' à la fréquence 0,6Hz (Pa) | Viscosité au repos (Pa.s) | Fréquence de crossover (Hz) |
| / | / | / | SF1 | Oui | 3 | ND | 0,04Hz |
| / | / | / | SF2 | Oui | 1 | ND | 0,3Hz |
| / | / | / | SF3 | Oui | 0,8 | 7 | 0,4Hz |
| / | / | / | SF4 | Oui | 1 | 1 | 0,6Hz |
| VS1 | 115 | 210 | SF2 | Non | 10 | 20 | 1,9Hz |
| VS2 | 0 | 7 | SF2 | Non | 0 | 20 | 0,8Hz |
| VS3 | 115 | 830 | SF2 | Oui | 9 | 90 | Pas de croisement |
| 8-147D | 130 | 2580 | SF2 | Oui | 89 | 11100 | Pas de croisement |
| 14-63B7 | 150 | 5400 | SF4 | Oui | 41 | 1400 | Pas de croisement |
| 8-145D | 30 | 4200 | SF1 | Oui | 12 | 1500 | Pas de croisement |
| 8-145C | 60 | 5600 | SF1 | Oui | 55 | 6650 | Pas de croisement |
| 8-145A | 520 | 8200 | SF1 | Oui | 450 | 64500 | Pas de croisement |

Il ressort de cet exemple que :

- le liquide synovial de patients atteints d'ostéoarthrose au niveau du genou est un gel de propriétés rhéologiques faibles ; les viscosuppléments et les formulations à base de chitosane succinamide ont des propriétés supérieures ;
- les viscosuppléments à base d'HA voient leurs propriétés rhéologiques chuter en les diluant avec le SF ;
- les formulations à base de CSS conservent leurs propriétés de gel après dilution dans le liquide synovial ;
- les formulations à base de CSS ont des propriétés rhéologiques renforcées par mélange avec le liquide synovial d'un adulte volontaire atteint d'ostéoarthrose, contrairement aux viscosuppléments commerciaux à base d'HA réticulé ou non. Il se produit probablement une interaction avec les composants du liquide synovial, par exemple l'acide hyaluronique ou les aggrécans.

**Exemple 19** - **Reproductibilité des formulations à base de chitosane succinamide au départ de plusieurs lots de chitosane succinamide**

Des solutions de chitosane succinamide de concentration variable sont préparées comme décrit précédemment, au départ de différents lots de CSS de degré de substitution similaires de 19 à 28% (références CSS22, CSS24, CSS25, CSS29 et CSS30) et de masse moléculaire similaires (médium), sans GP. On mesure leurs propriétés rhéologiques à l'aide à l'aide du rhéomètre « Discovery Hybrid DHR-2 » (TA Instrument) à 37°C selon les méthodes décrites précédemment, avant et après traitement par autoclave (tableaux 24a et 24b).

**Tableau 24a** - Avant autoclave

| Réf CSS | DS (mol%) | Formulation (référence) | Fréquence de crossover (Hz) | G' à la fréquence 0,6Hz (Pa) | Viscosité au repos (Pa.s) |
|---|---|---|---|---|---|
| CSS25 | 20 | 2 (008-147E) | Pas de croisement | 170 | 12300 |
| CSS29 | 20 | 5 (016-013A) | | 159 | 8000 |
| CSS30 | 28 | 1 | ND | ND | ND |
| CSS24 | 20 | 3 (016-006B) | Pas de croisement | 75 | 3600 |
| CSS22 | 19 | 4a (016-006C1) | | 95 | 4000 |
| | | 4b (016-008A) | | 106 | 4800 |

**Tableau 24b** - Après autoclave

| Formulation (référence) | Fréquence de crossover | G' à la fréquence 0,6Hz (Pa) | Viscosité au repos (Pa.s) | Injectabilité (aiguille 27G, N) |
|---|---|---|---|---|
| 2 | ND | ND | ND | ND |
| 5 (016-014A) | Pas de croisement | 129 | 6745 | ND |
| 1 | ND | ND | ND | 9N |
| 3 (016-010B) | | 133 | 8400 | 9N |
| 4a (016-010C) | Pas de croisement | 167 | 7800 | 10N |
| 4b (016-010D) | | 109 | 5500 | 13N |

On observe de cet exemple que :

- Le traitement à l'autoclave conserve le caractère de gel, le module d'élasticité et la viscosité au repos des formulations ;

- On peut préparer de manière reproductible des formulations dont les propriétés rhéologique et d'injectabilité sont similaires, au départ de plusieurs lots de CSS différents, provenant eux-même de chitosanes de lots différents dans la gamme de masse moléculaire « médium ».

**Exemple 20** - **Injectabilité de formulations de chitosane succinamide et de viscosuppléments commerciaux**

On mesure la force nécessaire pour injecter différentes formulations à base de chitosane succinamide et de viscosuppléments commerciaux au travers d'aiguilles pour injection de différents types et adaptées à l'injection intra-articulaire, à température ambiante selon la méthode décrite précédemment (tableau 25). Les tailles des différents types d'aiguilles sont définies à l'exemple 23.

**Tableau 25** - Force nécessaire pour injecter les formulations au travers d'aiguilles pour injection de différentes tailles (en Newton)

| Formulation | | Type d'aiguille (« normal-walled ») | | | |
|---|---|---|---|---|---|
| | | 21G | 22G | 25G | 27G |
| 017-008 | CSS 2% (sans GP) | 2,5 | / | 5 | 9 |
| 014-084 | CSS 1% / HA 1% (sans GP) | | 4 | 8 | 13 |
| 016-008B | CSS 2% (avec GP) | / | / | / | 13 |
| VS1 | VS1 | 9 | / | / | 20 |
| VS2 | VS2 | 2,5 | / | / | 9 |
| VS3 | VS3 | 4 | 7 | 15 | Difficilement injectable* |
| *présence de morceaux de gel | | | | | |

On comprend de cet exemple que les formulations de gels à base de chitosane succinamide sont facilement injectables au travers de fines aiguilles à température ambiante, tout en ayant des caractéristiques rhéologiques élevées à température physiologique de 37°C.

**Exemple 21** - **Susceptibilité à la dégradation in vitro en condition de stress oxydant**

Dans cet exemple, on met des formulations à base de chitosane succinamide et des viscosuppléments commerciaux en condition de stress oxydant in vitro pour simuler un environnement inflammatoire, selon un protocole adapté de la publication de Mendoza et al. (Carb Res 342, 96, 2007). Le protocole consiste à générer des radicaux libres hydroxyle par la réaction de Femton entre des ions cupriques et le peroxyde d'hydrogène, et de les mettre en présence des gels pendant 24 heures à température de 37°C. On prélève les gels et on mesure leur module d'élasticité G' et leur viscosité dynamique pour caractériser l'impact de l'incubation.

Brièvement, on place 800$\mu$g d'une formulation de gel à base de CSS ou d'un viscosupplément commercial à base de HA dans un tube à essai. On ajoute 100$\mu$g d'une solution d'EDTA de concentration 220$\mu$M et 70$\mu$g d'une solution de sulfate de suivre de concentration 1mM. On homogénéise le mélange, et on laisse incuber le mélange à 37°C durant 24 heures. On ajoute 30$\mu$g d'une solution de peroxyde d'hydrogène 0,03M, puis on prélève la solution à différents intervalles de temps, et on mesure les propriétés réhologiques à l'aide du rhéomètre Discovery HR-2 (TA) à 37°C selon les méthodes décrites précédemment.

On calcule le module d'élasticité relatif ($G'_R$) et la viscosité dynamique relative ($\eta_R$) selon les formules 1 et 2, respectivement :

$$\text{Formule 1 :} \quad G'_R = G' \text{ au temps } t \, / \, G' \text{ au temps zéro} \times 100 \quad \text{(tableau 26a)}$$

$$\text{Formule 2 :} \quad \eta_R = \eta \text{ au temps } t \, / \, \eta \text{ au temps zéro} \times 100 \quad \text{(tableau 26b)}$$

**Tableau 26a -** Module d'élasticité relatif (G'$_R$) des gels à une fréquence de 0,6 Hz (3,98 rad/s), en fonction du temps d'incubation en milieu oxydant

| Formulation (référence) | Type | 0 hr | 2 hr | 4 hr | 24 hr |
|---|---|---|---|---|---|
| 017-008 | Gel CSS (2%) | 100% | 127% | 125% | 92% |
| 014-084 | Gel CSS (1%) / HA (1%) | 100% | 99% | 42% | 51% |
| VS1 | Viscosupplément à base de HA non réticulé | 100% | 45% | 9% | 14% |
| VS3 | Viscosupplément à base de HA réticulé | 100% | 78% | 11% | 10% |

**Tableau 26b -** Viscosité dynamique relative ($\eta_R$) des gels en fonction du temps d'incubation en milieu oxydant

| Formulation (référence) | 0 hr | 2 hr | 4 hr | 24 hr |
|---|---|---|---|---|
| 017-008 | 100% | 129% | 128% | 92% |
| 014-084 | 100% | 99% | 46% | 51% |
| VS1 | 100% | 51% | 22% | 13% |
| VS3 | 100% | 86% | 14% | 10% |

Les formulations de gel à base de CSS de cet exemple sont significativement plus résistantes à la dégradation en milieu oxydant que les formulations commerciales à base d'acide hyaluronique réticulé (VS2) ou non réticulé (VS1), et elles conservent leur capacité à absorber les chocs (G') et leur pouvoir lubrifiant ($\eta$) pendant une durée prolongée, d'au moins 24 heures.

**Exemple 22** - **Osmolalité de solutions aqueuses de beta-glycérophosphate de sodium**

On dissout 10g de beta-glycérophosphate de sodium dans 100ml d'eau osmosée. La solution est agitée jusqu'à dissolution totale, puis diluée à différentes concentrations par ajout d'eau. L'osmolalité des solutions est mesurée selon la méthode décrote précédemment (tableau 27).

**Tableau 27 -** Osmolalité de solutions de beta-glycérophosphate de sodium (GP) dans l'eau

| Concentration en GP (g/100ml) | Osmolalité (mosmol/kg) |
|---|---|
| 2% | 155 |
| 4% | 296 |
| 6% | 438 |
| 7% | 512 |
| 8% | 590 |
| 9% | 658 |
| 10% | 745 |

On relève ainsi que l'osmolalité augmente avec la concentration de glycérophosphate dans de telles compositions. Ainsi une composition comprenant plus de 10 % de glycérophosphate présente une osmolalité allant au-delà de l'osmolalité désirée pour les applications envisagées dans la présente invention.
En particulier les publications de YUHUA Chang et al., '(« Preparation and properties of a novel thermosensitive N-trimethyl chitosane hydrogel, Polymer Bulletin, Springer, Berlin, DE, 63, 531) et de WU J. et al. (A thermo- and pH-sensitive hydrogel composed of quaternized chitosan/glycerophosphate, International Journal of Phamarmaceutics, Elsevier, BV, NL, 315, No 1-2, pages 1-11) décrivent des compositions comprenant plus de 10 % en masse de glycérophosphate. L'osmolalité de ces compositions est donc supérieure à 700 mosmol/kg et bien supérieure à 500 mosmol/kg.

On peut se reporter notamment au document relatif à la sécurité sanitaire du produit Nuflexxa® (Savient Pharmaceuticals, Inc. ; osmolalité de 258-381 mosm/kg) et de Negoro et al. (Effect of osmolarity on glycosaminoglycan production and cell metabolism of articular chondrocyte under three-dimensional culture system, Clinical and Experimental Rheumatology 2008, 26, 534-541) pour l'importance de la plage d'osmolalité. Une osmolalité d'environ 400mosm/kg est une valeur thérapeutiquement excellente car elle exerce une forte influence sur la prolifération de cellules telles que les chondrocytes, ostéocytes et les fibroblastes.

**Exemple 23** - **Diamètres des aiguilles pour injection**

Les tailles d'aiguilles pour injection sont définies par la norme ISO9262:1991 et l'amendement ISO9262:1991/Amd.1:2001(E). Le diamètre extérieur est fixe avec une tolérance dans la valeur (gamme min-max), tandis que le diamètre intérieur des aiguilles dépend de l'épaisseur des parois, qui sont d'épaisseur normale (« normal-walled »), fine (« thin-walled ») ou extra-fine (« extra-thin-walled ») selon les types d'aiguille et les applications. Le tableau 28 reprend les diamètres extérieurs (min/max) et intérieurs minimum des types d'aiguilles disponibles commercialement.

**Tableau 28** - Gamme d'aiguilles de différentes épaisseurs pour injection et tolérance des diamètres internes et externes, selon la norme ISO9262:1991/Amd.1:2001(E).

| Type (Gauge)* | Taille (mm) | Diamètre externe (μm) | | Diamètre interne (μm) | | |
| --- | --- | --- | --- | --- | --- | --- |
| | | | | « Normal-walled » or « Regular » | « Thin-walled » | « Extra-thin-walled » |
| | | Min | Max | Min | Min | Min |
| 32 | 0,23 | 229 | 241 | 89 | 105 | / |
| 31 | 0,25 | 254 | 267 | 114 | 125 | / |
| 30 | 0,30 | 298 | 320 | 133 | 165 | / |
| 29 | 0,33 | 324 | 351 | 133 | 190 | / |
| 28 | 0,36 | 349 | 370 | 133 | 190 | / |
| 27 | 0,40 | 400 | 420 | 184 | 241 | / |
| 26 | 0,45 | 440 | 470 | 232 | 292 | / |
| 25 | 0,5 | 500 | 530 | 232 | 292 | / |
| 24 | 0,55 | 550 | 580 | 280 | 343 | / |
| 23 | 0,60 | 600 | 673 | 317 | 370 | 460 |
| 22 | 0,70 | 698 | 730 | 390 | 440 | 522 |
| 21 | 0,80 | 800 | 830 | 490 | 547 | 610 |
| 20 | 0,90 | 860 | 920 | 560 | 635 | 687 |
| 19 | 1,10 | 1030 | 1100 | 648 | 750 | 850 |
| 18 | 1,20 | 1200 | 1300 | 790 | 910 | 1041 |
| *pour information | | | | | | |

**Exemple 24** - **Recommandations sur les types d'aiguilles pour injection**

Le tableau 29 liste les types d'aiguilles qui sont recommandés pour l'injection de différents produits commerciaux pour la réparation, le traitement ou le comblement, par exemple en intra-articulaire pour différents produits de viscosupplémentation commerciaux, en intra-dermal ou en sous-cutané pour des produits commerciaux de comblement du derme, en intra-dermal superficiel pour des produits commerciaux en vue de la régénération de la peau, ou encore en intra-camérulaire pour l'opthalmologie. Les diamètres externes et internes de ces types d'aiguilles sont indiqués à l'exemple 23.

**Tableau 29 -** Types d'aiguilles recommandées pour l'administration par injection, selon le site et le type d'injection et le produit considéré

| Produit | Type et site d'injection | Taille d'aiguille recommandée |
|---|---|---|
| Viscosupplément intra-articulaire (VS1) | Intra-articulaire / Genou | 19 à 21 Gauge |
| Viscosupplément intra-articulaire (VS2) | Intra-articulaire / Genou | 20 Gauge |
| Viscosupplément intra-articulaire (VS3) | Intra-articulaire / Genou | 18 à 20 Gauge |
| Viscosupplément intra-articulaire (VS4) | Intra-articulaire / Doigts | 19 à 25 Gauge |
| Réparation du cartilage par arthroscopie* | Arthroscopie / Genou, hanche | 18 Gauge |
| Comblement de ride à base d'acide hyaluronique réticulé | Intra-dermal / Visage | 25 à 27 Gauge |
| Comblement de ride à base de microparticules biorésorbables | Intra-dermal / Visage | 26 Gauge |
| Réjuvénation de la peau à base d'acide hyaluronique non réticulé | Sous-cutané ou intra-dermal / Visage, mains, cou | 30 à 32 Gauge** |
| Antibioprophylaxie | Intra-camérulaire / Chambre antérieure de l'oeil | 30G |
| *in : Stanish et al. 2006 ; **par exemple aiguille de type « pen needle » Micro-Fine™ Ultra 4mm (32G) commercialisée par BD. | | |

**Exemple 25** - **Test de stérilisation à la vapeur**

La solution de cet exemple est une solution comprenant 1,2% (m/m) de chitosane succinamide de degré de substitution 15% et de masse moléculaire « médium » (N° CSS16 du Tableau 2), 3,5% (m/m) de beta-glycérol phosphate de sodium, 0,4% (m/m) de D-mannitol et 11mmol/l de sodium acétate trihydrate (cf exemple 11). Cette solution est stérilisée selon le protocole suivant :

Placer 400 ml d'hydrogel dans une bouteille de Schott puis placer l'échantillon dans un autoclave (modèle Systec DX-23) avec un cycle liquide à 121°C pendant 20 minutes, selon une opération classique de stérilisation préconisée dans la norme ISO17665.

| Tableau 30. Caractéristiques d'une solution de chitosane succinamide avant et après stérilisation à la vapeur | | |
|---|---|---|
| | **Avant stérilisation** | **Après stérilisation** |
| **pH** | 7,5 | 7,51 |
| **Viscosité dynamique apparente** (Brookfield S18, 5 tours/min à 8°C) | 470 mPa.s | 370 mPa.s |
| **Osmolalité** | 488 mosm/kg | 507 mosm/kg |

Après stérilisation, l'hydrogel selon la présente invention présentait un pH, une viscosité et une osmolalité tout à fait convenables pour une utilisation chez l'être humain ou animal, notamment comme composition injectable à travers une aiguille. La mesure de l'osmolalité est réalisée comme décrit précédemment.

**Exemple 26** - **Cycle de transition sol-gel**

Les échantillons ont subi un cycle de stérilisation tel que décrit dans l'exemple 25. Plusieurs échantillons ainsi stérilisés ont été placés à une température initiale de 4°C. Ces échantillons sont fluides : lorsque l'on retourne le tube, on constate visuellement que la solution est fluide à l'intérieure du tube. Les échantillons ont ensuite subi un cycle de gélification en les plaçant dans une étuve une heure à 37 °C. Les échantillons sont gélifiés. La présence d'un gel est constatée visuellement par un renversement du tube : le gel ne s'écoule pas.

Les mêmes échantillons subissent un refroidissement et sont maintenus une heure au minimum à 4°C. Les échantillons sont fluides : lorsque l'on retourne le tube, on constate visuellement que la solution est fluide à l'intérieure du tube. Les échantillons peuvent donc que subir plusieurs transitions sol-gel et présentent donc une réversibilité de la transition sol-gel.

Des échantillons ont subi 3 cycles de gélation-dégélation.

**Exemple 27- Test de gélification après stérilisation**

| Tableau 31. | | | | | |
|---|---|---|---|---|---|
| | | Gélification avant stérilisation | | Gélification après stérilisation | |
| Descriptif | pH | Gélification à 37°C | Injectabilité | Gélification à 37°C | Injectabilité |
| Solution CsS/GP sodium | 8,2 | OK | OK | OK | OK |
| Solution CsS hydrogel Na | 7,6 | OK | OK | OK | OK |
| Solution CsS témoin (pas de stérilisation) | 7,55 | OK | OK | OK | OK |
| Solution CsS/GP sodium | 7,92 | OK | OK | OK | OK |
| Solution CsS témoin (pas de stérilisation) | 7,55 | OK | OK | OK | OK |

L'injectabilité est mesurée comme décrit précédemment. Les solutions se présentent bien sous la forme d'un gel à 37°C.

**Exemple 28** - **Test de contamination microbienne**

Des compositions thermogélifiables de l'invention ont été introduites dans des seringues en plastique à usage médical unique, puis stérilisée à la vapeur selon la stérilisation décrite précédemment. Ensuite des tests de croissance microbienne ont été réalisés :
Les seringues stérilisées comprenant les compositions thermogélifiables sont placées soit sur une gélose Columbia avec 5% sang de mouton (gélose COS) et gélose Chocolat PolyViteX (gélose PVX), dans des conditions aérobies à 35°C avec 5 % de CO2, soit dans des tubes de thioglycolate (THIO) à 35 °C sous-cultivés dans la gélose Columbia. Aucune contamination microbienne n'est décelée : les tests de croissance sont tous négatifs.
Le cycle de stérilisation est donc adapté pour stériliser des compositions thermogélifiables selon l'invention, par exemple pour un usage injectable chez l'être humain ou animal.

L'invention concerne donc les items suivants :

1.- Composition thermogélifiable stérilisée comprenant un chitosane substitué présentant des unités N-acétyl-D-glucosamine, des unités D-glucosamine, et des unités D-glucosamine substituées autres que les unités N-acétyl-D-glucosamine et éventuellement des unités N-acétyl-D-glucosamine substituées.

2.- Composition thermogélifiable stérilisée, selon l'item 1, caractérisée en ce que la transition sol-gel est réversible.

3.- Composition thermogélifiable stérilisée, selon l'item 1 ou 2, caractérisée en ce qu'elle est stérilisée à la vapeur.

4.- Composition, selon l'un quelconque des items 1 à 3, caractérisée en ce qu'elle n'est pas gélifiée après stérilisation.

5.- Solution stérilisée, ladite solution étant constituée de ou comprenant une composition thermogélifiable selon l'un quelconque des items 1 à 4.

6.- Procédé de stérilisation d'une composition thermogélifiable, caractérisé en ce qu'il comprend au moins les étapes suivantes :

- placer une composition thermogélifiable comprenant un chitosane substitué présentant des unités N-acétyl-D-glucosamine, des unités D-glucosamine, et des unités D-glucosamine substituées autres que les unités N-acétyl-D-glucosamine dans l'enceinte d'un autoclave ;
- élever et maintenir la température de l'enceinte de l'autoclave à une température de stérilisation et pendant une durée de stérilisation, la température et la durée de stérilisation étant suffisantes pour stériliser la composition thermogélifiable ;
- diminuer la température de l'enceinte de l'autoclave ;
- sortir la composition thermogélifiable stérilisée de l'enceinte de l'autoclave.

7.- Procédé, selon l'item 6, caractérisé en ce que la température de l'enceinte est maintenue à une température supérieure à 60°C, de préférence supérieure à 100°C, de préférence supérieure à 120°C.

8.- Procédé, selon l'item 7 ou 8, caractérisé en ce que la durée de stérilisation est d'au moins 3 minutes, de préférence d'au moins 10 minutes, de préférence d'au moins 15 minutes et encore de préférence d'au moins 20 minutes.

9.- Procédé, selon l'un quelconque des items 6 à 8, caractérisé en ce qu'il comprend un ou plusieurs cycles de stérilisation à la vapeur.

10.- Composition injectable caractérisée en ce qu'elle comprend ou consiste en une composition thermogélifiable selon l'un quelconque des items 1 à 6 ou susceptible d'être obtenu selon un procédé tel que défini à l'une quelconque des items 7 à 9.

11.- Composition injectable, selon l'item 10, caractérisée en ce qu'elle est utilisée comme composition pharmaceutique injectable ou dispositif médical injectable ou implantable.

12.-Composition selon l'item 11, pour une utilisation pour une méthode de traitement thérapeutique comprenant l'injection par voie sous-cutanée, intradermique, intraoculaire, ou intra-articulaire, de ladite composition, ou pour une utilisation pour la réparation ou le comblement d'au moins un tissu corporel nécessitant une réparation ou un comblement.

13.- Composition, selon l'item 12, caractérisée en ce que le tissu corporel est choisi parmi les tissus appartenant aux cordes vocales, muscles, ligaments, tendons, cartilages, organes sexuels, os, articulations, yeux, derme, ou l'une quelconque de leurs combinaisons, et plus particulièrement aux joints articulaires.

14.-Composition selon l'item 12 ou 13, pour son utilisation dans une méthode de traitement d'une arthrose, ou la réparation d'un défaut de cartilage, par exemple par injection dans le fluide synovial ou après mélange avec le sang et implantation dans le cartilage.

15.- Dispositif médical, par exemple implant médical, caractérisé en ce qu'il comprend ou consiste en une composition telle que définie à l'un quelconque des items 10 à 14.


## Revendications

1. Composition thermogélifiable stérilisée injectable ou implantable dans le corps humain ou animal, ladite composition comprenant un chitosane substitué présentant des unités N-acétyl-D-glucosamine, des unités D-glucosamine, et des unités D-glucosamine substituées autres que les unités N-acétyl-D-glucosamine et éventuellement des unités N-acétyl-D-glucosamine substituées, ladite composition comprenant une concentration de chitosane substitué inférieure à 4%.

2. Composition, selon la revendication 1, **caractérisée en ce qu'**elle comprend une transition sol-gel réversible.

3. Composition, selon la revendication 1 ou 2, **caractérisée en ce qu'**elle est sous forme de gel aqueux.

4. Composition, selon l'une quelconque des revendications 1 à 3, **caractérisée en ce qu'**elle ne comprend pas de sel de glycérol phosphate.

5. Composition, selon l'une quelconque des revendications 1 à 4, **caractérisée en ce qu'**elle comprend un sucre réducteur, par exemple le mannitol ou le sorbitol.

6. Composition, selon l'une quelconque des revendications 1 à 5, **caractérisée en ce qu'**elle est sous forme de gel avant utilisation et à température d'utilisation dans le corps humain ou animal à pH neutre ou à pH physiologique, avec une osmolalité physiologique convenable pour l'utilisation envisagée.

7. Composition, selon l'une quelconque des revendications 1 à 6, **caractérisée en ce qu'**elle présente une osmolalité de 200 à 650 mosm/kg.

8. Composition, selon l'une quelconque des revendications 1 à 7, **caractérisée en ce qu'**elle présente un pH supérieur ou égal à 7, par exemple supérieur ou égal à 7,1.

9. Composition, selon l'une quelconque des revendications 1 à 8, pour son utilisation comme composition pharmaceutique injectable ou dispositif médical injectable ou implantable.

10. Composition, selon l'une quelconque des revendications 1 à 9, pour son utilisation dans une méthode de traitement thérapeutique comprenant l'injection par voie sous-cutanée, intradermique, intraoculaire, ou intra-articulaire, de ladite composition, ou pour une utilisation pour la réparation ou le comblement d'au moins un tissu corporel nécessitant une réparation ou un comblement.

11. Composition, selon l'une quelconque des revendications 1 à 9, pour son utilisation dans une méthode de traitement d'une arthrose, ou la réparation d'un défaut de cartilage, par exemple par injection dans le fluide synovial ou après mélange avec le sang et implantation dans le cartilage.

12. Dispositif d'injection **caractérisé en ce qu'**il comprend une composition telle définie selon l'une quelconque des revendications 1 à 11.

13. Dispositif médical, par exemple implant médical, **caractérisé en ce qu'**il comprend ou consiste en une composition telle définie selon l'une quelconque des revendications 1 à 11.

14. Article ou packaging comprenant un ou plusieurs dispositifs selon la revendication 12 ou 13.

15. Procédé de préparation d'une composition selon l'une quelconque des revendications 1 à 11, comprenant :

  - la dissolution d'un chitosane substitué dans une solution aqueuse, de préférence de l'eau, éventuellement tamponnée;
  - l'ajustement éventuel du pH à un pH physiologique;
  - l'ajustement éventuel de l'osmolalité de la composition ;
  - l'ajustement éventuel de la viscosité de la composition.

16. Procédé de préparation d'un dispositif selon la revendication 12 ou 13, comprenant :

  - la dissolution d'un chitosane substitué dans une solution aqueuse, de préférence de l'eau, éventuellement tamponnée ;
  - l'ajustement éventuel du pH à un pH physiologique ;
  - l'ajustement éventuel de l'osmolalité de la composition ;
  - l'ajustement éventuel de la viscosité de la composition.
  - le remplissage d'un dispositif d'injection, comme par exemple une seringue, avec la composition obtenue ;
  - le dispositif d'injection, comme par exemple une seringue, subit ensuite une stérilisation à la vapeur,
  - le dispositif, par exemple une seringue, peut ensuite être emballé.

**Patentansprüche**

1. Sterilisierte thermogelierbare Zusammensetzung, die in den menschlichen oder tierischen Körper injizierbar oder implantierbar ist, wobei die Zusammensetzung ein substituiertes Chitosan umfasst, N-Acetyl-D-glucosamin-Einheiten, D-Glucosamin-Einheiten und andere substituierte D-Glucosamin-Einheiten als die N-Acetyl-D-glucosamin-Einheiten und gegebenenfalls substituierte N-Acetyl-D-glucosamin-Einheiten umfasst, wobei die Zusammensetzung eine substituierte Chitosan-Konzentration von weniger als 4% umfasst.

2. Sterilisierte thermogelierbare Zusammensetzung nach Anspruch 1, **dadurch gekennzeichnet, dass** der Sol-Gel-Übergang reversibel ist.

3. Zusammensetzung nach Anspruch 1 oder 2, **dadurch gekennzeichnet, dass** sie in Form eines wässrigen Gels vorliegt.

4. Zusammensetzung nach einem der Ansprüche 1 bis 3, **dadurch gekennzeichnet, dass** sie kein Glycerinphosphatsalz enthält.

5. Zusammensetzung nach einem der Ansprüche 1 bis 4, **dadurch gekennzeichnet, dass** sie einen reduzierenden Zucker, beispielsweise Mannit oder Sorbit, enthält.

6. Zusammensetzung nach einem der Ansprüche 1 bis 5, **dadurch gekennzeichnet, dass** sie in Form eines Gels vor der Verwendung und bei einer Gebrauchstemperatur im menschlichen oder tierischen Körper bei neutralem pH-Wert oder physiologischem pH-Wert mit einer physiologischen Osmolalität vorliegt, die für die vorgesehene Verwendung geeignet ist.

7. Zusammensetzung nach einem der Ansprüche 1 bis 6, **dadurch gekennzeichnet, dass** sie eine Osmolalität von 200 bis 650 mosm/kg aufweist.

8. Zusammensetzung nach einem der Ansprüche 1 bis 7, **dadurch gekennzeichnet, dass** sie einen pH-Wert größer oder gleich 7, beispielsweise größer oder gleich 7,1, aufweist.

9. Zusammensetzung nach einem der Ansprüche 1 bis 8 zur Anwendung als injizierbare pharmazeutische Zusammensetzung oder injizierbares oder implantierbares medizinische Vorrichtung.

10. Zusammensetzung nach einem der Ansprüche 1 bis 9 zur Anwendung in einem therapeutischen verfahren, umfassend eine subkutane, intradermale, intraokulare oder intraartikuläre Injektion der Zusammensetzung oder zur Anwendung bei der Reparatur oder Auffüllen von mindestens einem Körpergewebe, das eine Reparatur oder ein Augfüllen benötig.

11. Zusammensetzung nach einem der Ansprüche 1 bis 9 zur Anrwendung in einem Verfahren zur Behandlung einer Arthrose oder Reparatur eines Knorpeldefekts, beispielsweise durch Injektion in die Synovialflüssigkeit oder nach dem Mischen mit Blut und Implantieren in den Knorpel.

12. Injektionsvorrichtung, **dadurch gekennzeichnet, dass** sie eine wie in einem der Ansprüche 1 bis 11 definierte Zusammensetzung umfasst oder aus einer solchen besteht.

13. Medizinische Vorrichtung, zum Beispiel medizinisches Implantat, **dadurch gekennzeichnet, dass** sie eine wie in einem der Ansprüche 1 bis 11 definierte Zusammensetzung umfasst oder aus einer solchen besteht.

14. Gegenstand oder Verpackung mit einer oder mehreren Vorrichtungen nach Anspruch 12 oder 13.

15. Verfahren zur Herstellung einer Zusammensetzung nach einem der Ansprüche 1 bis 11, umfassend:

   - die Auflösung eines substituierten Chitosans in einer wässrigen Lösung, vorzugsweise Wasser, gegebenenfalls gepuffert;
   - die eventuelle Einstellung des pH-wert auf ein physiologisches pH-wert;
   - die eventuelle Einstellung der Osmolalität der Zusammensetzung;
   - die eventuelle Einstellung der Viskosität der Zusammensetzung.

16. Verfahren zur Herstellung einer Vorrichtung nach Anspruch 12 oder 13, umfassend :

   - die Auflösung eines substituierten Chitosans in einer wässrigen Lösung, vorzugsweise Wasser, gegebenenfalls gepuffert;
   - die eventuelle Einstellung des pH-Wertes auf einen physiologischen pH-Wert;

- die eventuelle Einstellung der Osmolalität der Zusammensetzung;
- die eventuelle Einstellung der Viskosität der Zusammensetzung;
- Füllen einer Injektionsvorrichtung, wie z.B. einer Spritze, mit der erhaltenen Zusammensetzung;
- Dampfsterilisierung der Injektionsvorrichtung, wie z.B. eine Spritze,
- verpackung der Injektionsvorrichtung, zum Beispiel eine Spritze.

**Claims**

1. Sterilized thermogelling composition injectable or implantable in a human or animal body, said composition comprising a substituted chitosan having N-acetyl-D-glucosamine units, D-glucosamine units, and substituted D-glucosamine units other than the N-acetyl-D-glucosamine units and optionally substituted N-acetyl-D-glucosamine units, said composition comprising a concentration of substituted chitosan lower than 4%.

2. Composition, according to claim 1, **characterized in that** the sol-gel transition is reversible.

3. Composition, according to claim 1 or 2, **characterized in that** it is in the form of an aqueous gel.

4. Composition, according to any one of claims 1 to 3, **characterized in that** it does not comprise a salt of glycerol phosphate.

5. Composition, according to any one of claims 1 to 4, **characterized in that** it comprises a reducing sugar, for example mannitol or sorbitol.

6. Composition, according to any one of claims 1 to 5, **characterized in that** it is in the form of an aqueous gel before use and at temperature of use in the human or animal body at neutral pH or at physiological pH, with a physiological osmolality suitable for considered use.

7. The composition, according to any one of claims 1 to 7, **characterized in that** it presents an osmolality of 200 to 650 mosm/kg.

8. The composition, according to any one of claims 1 to 4, **characterized in that** it presents a pH higher than or equal to 7, for example higher than or equal to 7.1.

9. The composition, according to any one of claims 1 to 8, for use as an injectable or implantable pharmaceutical composition or medical device.

10. The composition, according to any one of claims 1 to 9, for use in a method of therapeutic treatment comprising injecting via a sub-cutaneous, intradermal, intraocular, or intra-articular route, said composition, or for use for repairing or filling at least one body tissue requiring repair or filling.

11. The composition, according to any one of claims 1 to 9, for use in a method for treating arthrosis, or repairing a cartilage defect, for example by injection into the synovial fluid or after mixture with blood and implantation in the cartilage.

12. Injectable device **characterized in that** it comprises or consists in a composition as defined in any of claims 1 to 11.

13. Medical device, for example a medical implant, **characterized in that** it comprises or consists in a composition as defined in any of claims 1 to 11.

14. Article or packaging comprising one or more devices according to claim 12 or 13.

15. Process for preparing a composition according to any of claims 1 to 11, comprising:

- dissolution of a substituted chitosan in an aqueous solution, preferably water, optionally buffered;
- optional adjustment of the pH to a physiological pH;
- optional adjustment of the osmolality of the composition;
- optional adjustment of the viscosity of the composition.

16. Process for preparing a device according to claim 12 or 13, comprising:

- dissolution of a substituted chitosan in an aqueous solution, preferably water, optionally buffered;
- optional adjustment of the pH to a physiological pH;
- optional adjustment of the osmolality of the composition;
- optional adjustment of the viscosity of the composition;
- filling the injection device, such as for example a syringe, with the obtained composition;
- the injection device is then subject to steam sterilization,
- the device, for example a syringe, may be then packaged.

FIG.1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2361640 A
- US 6344488 B
- WO 2010142507 A
- US 7838643 B
- US 3953608 A
- WO 03068824 A
- EP 1483299 A
- US 7556946 B

**Littérature non-brevet citée dans la description**

- **JARRY et al.** Effects of steam sterilization on thermogelling chitosan-based gels. *J Biomed Mater Res (Appl Biomater),* 2011, vol. 58, 127-135
- **CHENGDONG JI et al.** la stérilisation de chitosane en présence de glycérol phosphate. *Sterilization-free chitosan hydrogels for controlled drug release, Materials Letters,* 2012, vol. 72, 110-112
- **JARRY et al.** Irradiating or autoclaving chitosan/polyol solutions: effect on thermogelling chitosan-β-glycerophosphate systems. *Chem Pharm Bull,* 2002, vol. 50 (10), 1335-1340
- *CHEMICAL ABSTRACTS,* 9012-76-4
- **RINAUDO et al.** *Int. J. Biol. Macromol,* 1993, vol. 15, 281-285
- *Clin Orthop Relat Res,* 1988, vol. 235, 289-95
- *Biochem Biophys Res Comm,* 2012, vol. 422, 455-461
- *J Bone Joint Surg Br,* 1959, vol. 41-B (2), 388-400
- *Acta Orthop Scand,* 1969, vol. 40, 634-641
- *Clin Rheumatol,* 2006, vol. 25, 886-888
- **LAVERTY et al.** *Osteoarthr Cartil,* 2010
- **EDOUARD et al.** *Phys Ther Sport,* 2013
- **MAINIL-VARLET et al.** *Cartilage,* 2013
- **OPRENYESZK et al.** *Osteoarthr Cartil,* 2013
- **MENDOZA et al.** *Carb Res,* 2007, vol. 342, 96
- reparation and properties of a novel thermosensitive N-trimethyl chitosane hydrogel. **YUHUA CHANG et al.** Polymer Bulletin. Springer, vol. 63, 531
- A thermo- and pH-sensitive hydrogel composed of quaternized chitosan/glycerophosphate. **WU J. et al.** International Journal of Phamarmaceutics. Elsevier, vol. 315, 1-11
- **NEGORO et al.** Effect of osmolarity on glycosaminoglycan production and cell metabolism of articular chondrocyte under three-dimensional culture system. *Clinical and Experimental Rheumatology,* 2008, vol. 26, 534-541